# EUROPÄISCHE PATENTSCHRIFT

(11) **EP 4 039 636 B1**
(45) Veröffentlichungstag und Bekanntmachung des Hinweises auf die Patenterteilung: **01.11.2023**
(21) Anmeldenummer: 22153312.8
(22) Anmeldetag: 25.01.2022
(51) Int. Cl.: C01B 3/00, C01B 3/22, C02F 11/10, C12M 1/107, C25B 1/04, C02F 11/04, C02F 11/121, C12M 1/00

(54) **VERFAHREN ZUR ERZEUGUNG VON WASSERSTOFF**
METHOD FOR PRODUCING HYDROGEN
PROCÉDÉ DE PRODUCTION D'HYDROGÈNE

(30) Priorität: 29.01.2021 DE 102021102127
(43) Veröffentlichungstag der Anmeldung: 10.08.2022
(73) Patentinhaber: N-ERGIE Aktiengesellschaft, 90429 Nürnberg (DE)
(72) Erfinder: FLEISCHER, Patrick, 90429 Nürnberg (DE)
(74) Vertreter: Ellwanger, Arndt

(56) Entgegenhaltungen:
- EP-A1- 3 415 609
- EP-A1- 3 757 193
- DE-A1- 2 909 676
- DE-A1-102013 223 591
- US-B2- 10 396 388

## Beschreibung

Die vorliegende Erfindung betrifft ein Verfahren zur Erzeugung von Wasserstoff, wobei Wasserstoff aus einem mit Wasserstoff beladenen Träger (T2) freigesetzt wird und zumindest eine Teilmenge der zur Wasserstofffreisetzung erforderlichen Energie durch die Verbrennung von Klärschlamm gewonnen wird. Dieser freigesetzte Wasserstoff kann wiederum zur Stromerzeugung eingesetzt werden, so dass die vorliegende Erfindung auch ein Verfahren zur Erzeugung von elektrischen Strom und gegebenenfalls von Wärme betrifft.

EP-A 3 415 609 betrifft ein Verfahren zum Speichern und Lagern von Wasserstoff, umfassend zwei Verfahrensschritte. Im ersten Verfahrensschritt wird der Wasserstoff gespeichert, indem Wasserstoff mit einem Träger (T1) in Kontakt gebracht wird, wodurch ein mit Wasserstoff beladener Träger (T2) erhalten wird. Der Wasserstoff wird dabei vorzugsweise chemisch, insbesondere im Rahmen einer Hydrierung, an den Träger (T1) gebunden. Im zweiten Verfahrensschritt erfolgt die Lagerung des mit Wasserstoff beladenen Träger (T2) in einer Vorrichtung (V1), die Bestandteil einer Biogasanlage ist oder war. Der im Träger (T2) gespeicherte Wasserstoff kann anschließend aus dem Träger (T2) wieder freigesetzt und nachfolgend zur Erzeugung von Strom und gegebenenfalls Wärme verwendet werden. Das in EP-A 3 415 609 beschriebene Verfahren kann in eine bestehende Biogasanlage integriert oder eine Biogasanlage kann entsprechend umgerüstet werden. Weiterhin ist dort ein Verfahren zur Erzeugung von elektrischem Strom und gegebenenfalls von Wärme offenbart, wobei zunächst das Verfahren zum Speichern und Lagern von Wasserstoff durchgeführt wird.

EP-A 3 415 610 betrifft ein Verfahren zur Umrüstung einer Biogasanlage in eine Anlage zum Lagern von Wasserstoff, umfassend mindestens zwei Verfahrensschritte. Im ersten Verfahrensschritt wird das Innere von mindestens einem Fermenter (F1) der Biogasanlage gereinigt. Im optionalen zweiten Verfahrensschritt wird gegebenenfalls mindestens eine Schutzauskleidung im Inneren des gereinigten Fermenters (F1) angebracht. Im dritten Verfahrensschritt wird ein mit Wasserstoff beladener Träger (T2) in den gereinigten Fermenter (F1) eingebracht und gelagert.

Biogasanlagen als solche sind bereits seit langem bekannt (siehe beispielsweise die freie Enzyklopädie Wikipedia unter dem Begriff "Biogasanlage"; https://de.wikipedia.org/wiki/biogasanlage; Version vom 19. April 2017). Eine Biogasanlage dient zur Erzeugung von Biogas durch Vergärung von Biomasse. Biogasanlagen werden in der Regel in landwirtschaftlichen Betrieben eingesetzt, wo beispielsweise tierische Exkremente (Gülle/Festmist) oder Energiepflanzen als Substrat eingesetzt werden. Als Nebenprodukt wird häufig ein als Gärrest bezeichneter Dünger produziert. Das bei der Biogasanlage als Hauptprodukt anfallende Biogas wird häufig zur dezentral gekoppelten Strom- und Wärmeerzeugung (Kraft-Wärme-Kopplung) in Blockheizkraftwerken genutzt. Dabei wird das Biogas, das gegebenenfalls noch getrocknet und/oder entschwefelt wird, einem Gasmotor zugeführt, der das Biogas in elektrischen Strom sowie gegebenenfalls in Wärme verwandelt. Der so produzierte Strom wird in das Netz eingespeist. Meistens erfolgt die Herstellung des elektrischen Stroms aus dem Biogas direkt vor Ort als Bestandteil einer Biogasanlage. Alternativ ist es auch möglich, dass das in einer Biogasanlage erzeugte Biogas auf Erdgasqualität gereinigt und als Biomethan (Bioerdgas) in das Erdgasnetz eingespeist wird.

Zentraler Bestandteil einer solchen Biogasanlage ist in der Regel ein Biogasreaktor, der auch als Fermenter bezeichnet wird (siehe auch Wikipedia; https://de.wikipedia.org/wiki/bioreaktor; Version vom 21. April 2017). In diesem Fermenter wird in Abhängigkeit von der eingebrachten Biomasse durch spezifische biologische Prozesse (Biokonversion, Biokatalyse) das Biogas erzeugt. In der Praxis gibt es zahlreiche Hersteller von solchen Reaktoren/Fermentern, die sich hinsichtlich Größe und Ausstattung sowie Art und Dimension der Zuleitungen der im Fermenter enthaltenen Einbauten, wie Rührwerke oder etwaige vorhandene Heizsysteme im Inneren des Fermenters, unterscheiden. Eine Biogasanlage kann auch zwei oder mehrere Fermenter enthalten. Weitere Fermenter werden auch als Nachgärer bezeichnet/eingesetzt. Weiterhin verfügen Biogasanlagen häufig über zahlreiche Vorrats- oder Lagerbehälter, um beispielsweise die zu vergärende Biomasse oder den Gärrückstand zu lagern. Die einzelnen Vorrichtungselemente einer Bioanlage sind in der Regel durch ein entsprechendes Leitungssystem sowie durch Pumpen miteinander verbunden.

Ein wesentliches Problem von Biogasanlagen ist deren wirtschaftliche Betreibung. Zumindest in Deutschland, aber auch in anderen europäischen Ländern sinngemäß, wird der Betrieb von Biogasanlagen im Rahmen der erneuerbaren Energien wirtschaftlich gefördert. Allerdings sind diese staatlichen Förderungen im Zusammenhang mit der Erzeugung von erneuerbaren Energien nicht unbegrenzt erhältlich, sondern werden in absehbarer Zeit, insbesondere im Zusammenhang mit dem Betrieb von Biogasanlagen, reduziert oder sogar auslaufen. Ohne staatliche Hilfe ist es für viele landwirtschaftliche Betriebe schwierig, insbesondere kleinere Biogasanlagen weiterhin wirtschaftlich zu betreiben. Ein weiteres Problem beim Betreiben von Biogasanlagen ist in der Gefahr von Gasleckagen bzw. dem Wärmeverlust zu sehen. Um das in der Regel brennbare Biogas zu handhaben bzw. zu lagern sind erhöhte Sicherheitsvorkehrungen notwendig.

DE-A 10 2012 005 023 offenbart eine Anordnung sowie ein Verfahren zur autonomen Bereitstellung von Elektrizität über Wasserstoff. Hierbei wird zunächst elektrischer Strom aus einer regenerativen Quelle bereitgestellt, der zur Herstellung von Wasserstoff aus Wasser in mindestens einem Elektrolyseur verwendet wird. Der so hergestellte Wasserstoff wird in einen ersten chemischen Reaktor überführt, der mindestens ein Substrat mit einem ausgedehnten pi-konjugierten System enthält, wobei in diesem ersten chemischen Reaktor eine zumindest teilweise Hydrierung des Substrats durchgeführt wird. Anschließend wird dieses zumindest teilweise hydrierte Substrat in einen Speichertank überführt. Aus diesem Speichertank wird das zumindest teilweise hydrierte Substrat wiederum in einen zweiten chemischen Reaktor überführt, wo eine Dehydrierung dieses Substrates unter Freisetzung von Wasserstoff erfolgt. Dieser im zweiten chemischen Reaktor erzeugte Wasserstoff wird in eine Anlage oder Maschine zur Wandlung des Wasserstoffs in elektrische Energie, vorzugsweise in eine Brennstoffzelle, überführt. Von dort wird elektrischer Strom an den Ort des Bedarfs abgegeben.

Das in DE-A 10 2012 005 023 beschriebene Verfahren bzw. die entsprechende Anlage eignet sich beispielsweise als Notstromaggregat oder an Orten, an denen ein öffentliches Stromnetz fehlt, sowie in Krisensituationen, wie Kriegen. Anwendungsgebiete können Krankenhäuser, chemische Anlagen, Computerzentralen oder auch Kernkraftwerke sein. Letztendlich wird bei diesem Verfahren Wasserstoff zwischengespeichert, was gegebenenfalls auch über einen längeren Zeitraum erfolgen kann, um ihn im Bedarfsfall zur Stromerzeugung einzusetzen. Der anfänglich bereitgestellte elektrische Strom, der aus regenerativen Quellen stammt, mit dem der Wasserstoff erzeugt wird, kann beispielsweise aus Wasserkraft, Windenergie, solarer Strahlung oder auch aus Biogas stammen. Die Durchführung des Verfahrens in Verbindung mit einer Klärschlammtrocknung ist in DE-A 10 2012 005 023 jedoch nicht offenbart.

Ein sinngemäßes Verfahren bzw. eine entsprechende Anordnung ist in DE-A 10 2011 121 704 offenbart. Die Anordnung (Vorrichtung) wird insbesondere zur Pufferung von Überschuss-Elektrizität in Gebäuden eingesetzt. Das entsprechende Verfahren dient zur Energiespeicherung in Gebäuden. Das Verfahren sowie die entsprechende Anordnung erfordern zwingend die Verwendung einer Brennstoffzelle zur Oxidation des in dem zweiten chemischen Reaktor freigesetzten Wasserstoffs unter Freisetzung von Energie.

Verfahren zum Trocknen und/oder Entsorgen von Klärschlamm, beispielsweise durch Verbrennung, sind ebenfalls bereits seit langem bekannt. Hintergrundinformationen zur Beschaffenheit von Klärschlamm sowie der Klärschlammentsorgung in der Bundesrepublik Deutschland können beispielsweise dem Artikel "Klärschlamm-Entsorgung in der Bundesrepublik Deutschland" (Stand: Oktober 2018, herausgegeben vom Bundesumweltamt, erhältlich über das Internet unter: www.umweltbundesamt.de/publikationen) entnommen werden. Weitere Informationen in diesem Zusammenhang können auch dem Artikel "Klärschlammtrocknung in Deutschland - Stand und Perspektiven" von Jürgen Geyer (Seiten 927-948; in Energie aus Abfall - Band 10; TK Verlag Karl Thome-Kozmiensky; Neuruppin 2013) entnommen werden.

Eine Verknüpfung der Klärschlammverbrennung mit der Freisetzung von Wasserstoff aus einem mit Wasserstoff beladenen Träger, wobei die hierfür erforderliche Energie durch die Verbrennung von Klärschlamm gewonnen wird, ist jedoch weder in den vorgenannten Artikeln noch sonst irgendwo bisher beschrieben worden. Sinngemäßes gilt auch für die Erzeugung von elektrischem Strom unter Verwendung des freigesetzten Wasserstoffs.

Die der vorliegenden Erfindung zugrunde liegende Aufgabe besteht in der Bereitstellung eines neuen Verfahrens zur Erzeugung von Wasserstoff bzw. zur Erzeugung von elektrischem Strom.

Gelöst wird die Aufgabe durch ein Verfahren zur Erzeugung von Wasserstoff, umfassend den folgenden Schritt a):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird.

Weiterhin wird die Aufgabe gelöst durch ein Verfahren zur Erzeugung von elektrischem Strom und gegebenenfalls von Wärme umfassend die nachfolgenden Schritte a) und b):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird,
b) Überführung des in Schritt a) erhaltenen Wasserstoffs in eine Vorrichtung (V2), wobei in der Vorrichtung (V2) Wasserstoff in elektrischen Strom und gegebenenfalls in Wärme umgewandelt wird.

Ein erster Vorteil des erfindungsgemäßen Verfahrens ist darin zu sehen, dass die bei einer Klärschlammverbrennung entstehende Wärme sinnvoll wiederverwendet werden kann, wobei in einem Dehydrierungsprozess aus einem mit Wasserstoff beladenen Träger, insbesondere bei einer Dehydrierung im LOHC-Verfahren, auf einfache Weise durch entsprechende Wärmezufuhr aus der Klärschlammverbrennung Wasserstoff aus dem entsprechenden Träger freigesetzt werden kann. Bei einer solchen Dehydrierung, also der Freisetzung von Wasserstoff aus einem geeigneten Träger, handelt es sich um einen endothermen Vorgang, der die Einspeisung von großen Mengen an Energie, beispielsweise in Form von Wärme, erfordert. Die für diesen endothermen Vorgang erforderliche Energie muss somit nicht separat bzw. kostenintensiv erzeugt werden, sondern es kann die bei einer Klärschlammverbrennung generell freiwerdende Energie sinnvoll eingesetzt werden.

Dieser freigesetzte Wasserstoff kann wiederum auf einfachem Wege zur Erzeugung von elektrischem Strom eingesetzt werden. Die bei der Erzeugung von elektrischen Strom unter Verwendung von Wasserstoff in der Regel zusätzlich freiwerdende Wärme kann im Bedarfsfall in den vorausgegangenen Verfahrensschritte zur Freisetzung von Wasserstoff aus einem mit Wasserstoff beladenen Träger rückgeleitet werden, was einen weiteren Vorteil des erfindungsgemäßen Verfahrens darstellt. Dies ist insbesondere dann vorteilhaft, sofern sich die entsprechenden Vorrichtungen zur Freisetzung von Wasserstoff aus dem Träger einerseits und zur Stromerzeugung andererseits in räumlicher Nähe zueinander befinden.

Ein weiterer Vorteil des erfindungsgemäßen Verfahrens ist darin zu sehen, dass es sich problemlos mit weiteren Verfahrensschritten zur Speicherung von Energie und/oder elektrischen Strom oder Wärme kombinieren lässt. Beispielsweise kann das erfindungsgemäße Verfahren Bestandteil eines Kreislaufs zur Speicherung von Energie bzw. elektrischen Strom sein, insbesondere in Verbindung mit einem LOHC-Verfahren, so dass Energie über einen längeren Zeitraum sicher zwischengespeichert werden kann.

Dieser Vorteil kann sehr anschaulich anhand der nachfolgenden Fallkonstellation verdeutlicht werden. Beispielsweise wird in günstigen Zeiten ein Überschuss an Energie in Form von elektrischem Strom erzeugt. Denkbar sind hier saisonal verteilte windreiche Tage im Zusammenhang mit der Erzeugung von elektrischem Strom durch Windenergie bzw. sonnenreiche Tage, insbesondere während des Sommerhalbjahres, zur Erzeugung von elektrischem Strom, beispielsweise über Photovoltaikanlagen oder Sonnenkollektoren. Der jeweilige Überschuss an Energie, insbesondere an elektrischem Strom, kann beispielsweise in Wasserstoff umgesetzt werden. Erfindungsgemäß wird Wasserstoff, der vorzugsweise aus elektrischen Strom gewonnen werden kann, durch Inkontaktbringen mit einem geeigneten Träger sicher gespeichert, sodass der Wasserstoff bzw. indirekt die Energie über einen längeren Zeitraum gelagert werden kann. Im Bedarfsfall, also bei einem plötzlich eintretenden erhöhten bzw. kontinuierlichen Bedarf an elektrischer Energie, kann der so zwischengespeicherte Wasserstoff bzw. die zwischengespeicherte Energie wieder in elektrischen Strom oder gegebenenfalls in Wärme rückgewandelt bzw. umgewandelt werden.

Ein weiterer Vorteil des erfindungsgemäßen Verfahrens ist darin zu sehen, dass die bei der vorstehend beschriebenen saisonalen Speicherung von elektrischem Strom in Folge der exothermen Einspeicherung von Wasserstoff freiwerdende Energie wiederverwendet werden kann. Dies ist insbesondere dann ein großer Vorteil, wenn das Ein- und Ausspeichern von Wasserstoff, insbesondere bei dem nachstehend beschriebenen bevorzugten LOHC-Verfahren, entweder räumlich und/oder zeitlich getrennt voneinander erfolgen. Die Zwischenspeicherung der bei der Wasserstoffspeicherung freiwerdenden Energie ist generell schwierig bzw. mit relativ hohen Verlusten verbunden, insbesondere dann, wenn besagte Energie für einen längeren Zeitraum zwischengespeichert werden soll.

Die erfindungsgemäße Ankopplung einer Wasserstoffspeicherung gemäß nachfolgend definierten Schritt d), insbesondere im Rahmen eines LOHC-Prozesses, an eine Klärschlammtrocknung gemäß nachfolgend definierten Schritt e) bzw. an eine Kopplung an eine Klärschlammverbrennungsanlage ist somit in mehrfacher Hinsicht vorteilhaft. Einerseits kann (wie bereits beschrieben) die bei der Einspeicherung (Hydrierung) von Wasserstoff in einen geeigneten Träger, insbesondere eine LOHC-Flüssigkeit (bzw. in einem LOHC-Verfahren), entstehende Wärme im Prozess der Klärschlammtrocknung effizient genutzt werden. Der getrocknete Klärschlamm kann wiederum durch Verbrennung entsorgt werden, wodurch die im erfindungsgemäßen Verfahren in Schritt a) benötigte Wärme zur Freisetzung von Wasserstoff aus einem geeigneten Träger gewonnen werden kann, sodass sich der Energiekreislauf an dieser Stelle quasi schließt.

Das erfindungsgemäße Verfahren kann in den einzelnen nachfolgend beschriebenen Ausführungsformen in mehrfacher Hinsicht sowohl zeitlich versetzt und/oder räumlich getrennt voneinander durchgeführt werden. Letztendlich können die eingesetzten Träger, insbesondere LOHC-Flüssigkeiten, mehrfach mit Wasserstoff beladen und anschließend wieder entladen werden. Die jeweils benötigte oder freigesetzte Energie kann ebenfalls in einem Kreislauf und/oder räumlich bzw. zeitlich versetzt eingesetzt werden.

Ein weiterer Vorteil des erfindungsgemäßen Verfahrens ist darin begründet, dass in einer bevorzugten Ausführungsform die jeweilige Infrastruktur der in Deutschland bzw. Europa zahlreich vorhandenen Biogasanlagen verwendet werden kann, um das erfindungsgemäße Verfahren durchzuführen. Dadurch können die Anfangsinvestitionskosten gegenüber einem kompletten Neubau/dem Kauf einer kompletten neuen Anlage deutlich reduziert werden. Wesentliche Vorrichtungskomponenten jeder gängigen Biogasanlage können für das erfindungsgemäße Verfahren verwendet werden, ohne dass dabei Kosten im großen Umfang im Zusammenhang mit der Umrüstung einzelner Vorrichtungselemente einer Biogasanlage anfallen müssen. Weiterhin können Vorrichtungselemente, die entweder direkt zur Biogasanlage gehören oder ihr unmittelbar angeschlossen sind, wie beispielsweise ein Netzanschluss, ein Transformator, ein Blockheizkraftwerk oder ein darin enthaltener Gasmotor, auch ohne weitere Umrüstung direkt im erfindungsgemäßen Verfahren sinngemäß zum Betrieb bei einer Biogasanlage mit nachfolgender Stromerzeugung verwendet werden.

Ebenfalls ist es als vorteilhaft anzusehen, dass das erfindungsgemäße Verfahren auch parallel und/oder zeitversetzt zum herkömmlichen Betrieb einer Biogasanlage durchgeführt werden kann. So ist es denkbar, dass zusätzlich zum bisherigen "konventionellen Betrieb" einer Biogasanlage entsprechende Vorrichtungselemente ergänzt werden, um das erfindungsgemäße Verfahren parallel oder zeitversetzt zur eigentlichen Produktion von Biogas bzw. der nachfolgenden Strom- oder Wärmeerzeugung durchgeführt wird. Bei dieser Verfahrensvariante ist es weiterhin vorteilhaft, wenn der aus elektrischem Strom erzeugte Wasserstoff zumindest teilweise zur Erzeugung von Biogas, insbesondere im Rahmen einer Methanisierung, eingesetzt wird.

Alternativ ist es selbstverständlich auch möglich, dass eine bisher konventionell genutzte Biogasanlage vollumfänglich zur Durchführung des erfindungsgemäßen Verfahrens umgerüstet wird. Dies ist insbesondere im Fall von kleineren Biogasanlagen, die im landwirtschaftlichen Bereich betrieben werden, von Interesse, weil deren wirtschaftliche Betreibung aufgrund des zumindest in Deutschland mittlerweile erfolgten Endes der wirtschaftlichen Förderung solcher Biogasanlagen sehr schwierig bis unmöglich ist.

Im Rahmen der vorliegenden Erfindung hat der Begriff "in räumlicher Nähe" (sofern nachfolgend nicht anders definiert) die folgende Bedeutung: die jeweiligen Schritte werden auf demselben Betriebsgelände (oder auf einem angrenzenden Betriebsgelände) durchgeführt, vorzugsweise in einem (räumlichen) Abstand von maximal 1 km, mehr bevorzugt in einem Abstand von maximal 500 m, insbesondere in einem Abstand von maximal 100 m (bzw. die entsprechenden Vorrichtungen zur Durchführung dieser Schritte befinden sich in den entsprechenden Abständen). Sofern im Rahmen der vorliegenden Erfindung konkrete Zahlenwerte für einen Abstand angegeben sind, beziehen sich die jeweiligen Abstandsangaben auf die Luftlinie.

Im Rahmen der vorliegenden Erfindung hat der Begriff "räumlich getrennt voneinander" (sofern nachfolgend nicht anders definiert) die folgende Bedeutung: die jeweiligen Schritte werden nicht auf demselben Betriebsgelände (oder auf einem angrenzenden Betriebsgelände) durchgeführt, vorzugsweise in einem (räumlichen) Abstand von mindestens 5 km, mehr bevorzugt in einem Abstand von 10-50 km (bzw. die entsprechenden Vorrichtungen zur Durchführung dieser Schritte befinden sich in den entsprechenden Abständen). Erfindungsgemäß ist es prinzipiell möglich, dass die entsprechenden Schritte, die in einem räumlichen Abstand durchgeführt werden sollen, in deutlich größeren Abständen durchgeführt werden, beispielsweise in einem Abstand von mehreren 100 km oder sogar mehr als 1000 km. Sinngemäßes gilt für Begriffe wie "in räumlichem Abstand", "in räumlicher Distanz" oder "räumlich versetzt".

Im Rahmen der vorliegenden Erfindung hat der Begriff "in zeitlicher Nähe" (sofern nachfolgend nicht anders definiert) die folgende Bedeutung: die jeweiligen Schritte werden vorzugsweise parallel, also zur gleichen Uhrzeit, durchgeführt, insbesondere wenn sie auf demselben Betriebsgelände durchgeführt werden. Gegebenenfalls können die jeweiligen Schritte auch mit einer gewissen zeitlichen Verzögerung zueinander durchgeführt werden, vorzugsweise in einem (zeitlichen) Abstand von maximal 1 Tag, mehr bevorzugt in einem Abstand von maximal 6 Stunden, insbesondere in einem Abstand von maximal 1 Stunde (bzw. die entsprechenden Vorrichtungen zur Durchführung dieser Schritte werden im entsprechenden Zeitrahmen eingesetzt). Sinngemäßes gilt für Begriffe wie "in direkter zeitlicher Abfolge".

Im Rahmen der vorliegenden Erfindung hat der Begriff "zeitlich getrennt voneinander" (sofern nachfolgend nicht anders definiert) die folgende Bedeutung: die jeweiligen Schritte werden nicht parallel, also nicht zur gleichen Uhrzeit, durchgeführt, insbesondere werden sie nicht auf demselben Betriebsgelände durchgeführt. Stattdessen werden die jeweiligen Schritte mit einer zeitlichen Verzögerung zueinander durchgeführt, die mindestens einen Tag beträgt. Vorzugsweise beträgt der (zeitliche) Abstand zwischen der Durchführung der jeweiligen Schritte mindestens einen Monat, besonders bevorzugt mindestens drei Monate (bzw. die entsprechenden Vorrichtungen zur Durchführung dieser Schritte werden im entsprechenden Zeitrahmen eingesetzt). Erfindungsgemäß ist es prinzipiell möglich, dass die entsprechenden Schritte die in einem zeitlichen Abstand durchgeführt werden sollen, in deutlich größeren zeitlichen Abständen durchgeführt werden, beispielsweise in einem Abstand von mehreren Monaten oder sogar mehreren Jahren. Sinngemäßes gilt für Begriffe wie "in zeitlichem Abstand", "in zeitlicher Distanz" oder "zeitlich versetzt".

Nachfolgend wird das erfindungsgemäße Verfahren zur Erzeugung von Wasserstoff sowie die gegebenenfalls zusätzliche Erzeugung von elektrischem Strom und/oder von Wärme näher erläutert.

Der erste Gegenstand der vorliegenden Erfindung ist ein Verfahren zur Erzeugung von Wasserstoff, umfassend den folgenden Schritt a):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird.

Erfindungsgemäß wird in Schritt a) aus einem (mit Wasserstoff beladenen) Träger (T2) Wasserstoff freigesetzt. Durch die Freisetzung des Wasserstoffs wird zusätzlich der Träger (T1) erhalten. Die beiden Träger (T1) und (T2) zeichnen sich erfindungsgemäß dadurch aus, dass sie reversibel Wasserstoff binden (Träger (T1)) bzw. reversibel Wasserstoff freisetzen können (Träger (T2)). In anderen Worten ausgedrückt bedeutet dies, dass bei einer Wasserstofffreisetzung aus dem Träger (T2) der Träger (T1) gebildet wird, während aus dem Träger (T1) durch die Bindung mit Wasserstoff der Träger (T2) entsteht. Letztgenanntes wird auch als Wasserstoffspeicherung bezeichnet.

Erfindungsgemäß ist es somit bevorzugt, dass der in Schritt a) eingesetzte mit Wasserstoff beladene Träger (T2) aus einem Träger (T1) erhalten wird, wobei der Träger (T1) mit Wasserstoff in Kontakt gebracht wird, vorzugsweise dient der mit Wasserstoff beladene Träger (T2) als Wasserstoffspeicher.

Als Träger (T1) kann prinzipiell jeder beliebige, dem Fachmann bekannte Träger verwendet werden, der dazu geeignet ist, Wasserstoff in irgendeiner Form zu speichern. Weiterhin ist erfindungsgemäß der Träger (T1) dazu in der Lage, den Wasserstoff reversibel zu speichern. Dies bedeutet, dass aus dem mit Wasserstoff beladenen Träger (T2), der durch Inkontaktbringen des Wasserstoffs mit dem Träger (T1) erhalten wird, der Wasserstoff zu einem späteren Zeitpunkt wieder freigesetzt werden kann und dabei der ursprünglich eingesetzte Träger (T1) wiedergewonnen wird.

Das Speichern des Wasserstoffs kann beispielsweise so durchgeführt werden, dass Wasserstoff chemisch oder physisorptiv, vorzugsweise chemisch, an den Träger (T1) gebunden wird. Im Rahmen der vorliegenden Erfindung ist es jedoch erforderlich, dass das Speichern des Wasserstoffs reversibel möglich ist. Insbesondere Kohlendioxid, das chemisch mit Wasserstoff unter Erhalt von Methan reagieren kann, ist kein Träger (T1) im Sinne der vorliegenden Erfindung, weil aus Methan nicht direkt und/oder auf einfachem Wege reversibel Wasserstoff freigesetzt sowie Kohlendioxid wiedergewonnen werden kann. Folglich ist Methan auch kein mit Wasserstoff beladener Träger (T2) im Sinne der vorliegenden Erfindung.

Eine chemische Bindung des Wasserstoffs an den Träger (T1) kann beispielsweise dadurch erfolgen, dass der Träger (T1) ein Metallhydridspeicher ist, der ein aus einer Metalllegierung hergestelltes Material enthält, das Wasserstoff aufnimmt und chemisch bindet. Alternativ kann der Träger (T1) auch eine, vorzugsweise flüssige, organische Verbindung sein. Solche organischen Verbindungen werden auch als Wasserstoffträger oder LOHC (englisch für: liquid organic hydrogen carriers) bzw. organische Hydride bezeichnet. Solche Wasserstoffträger (LOHC) sind dem Fachmann bekannt und beispielsweise in DE-B 10 2013 223 589, EP-A 1 475 349 und DE-A 10 2012 005 023 beschrieben.

Diese Form der Wasserstoffspeicherung unter Verwendung der LOHCs hat den besonderen Vorteil, dass der LOHC-Träger (T1) in der Regel unter den verwendeten Prozessbedingungen in flüssiger Form vorliegt. Die physiochemischen Eigenschaften eines solchen LOHC-Trägers haben hohe Ähnlichkeit zu herkömmlichen flüssigen Kraftstoffen, sodass Pumpen zum Transport oder Behälter zur Lagerung aus dem Bereich der Kunststoff- und Printstofflogistik genutzt werden können. Die Wasserstoffspeicherung in chemisch gebundener Form in einer organischen Flüssigkeit wie LOHC erlaubt eine drucklose Lagerung bei Normalbedingungen über große Zeiträume ohne signifikanten Wasserstoffverlust. Sofern es sich bei dem Träger (T1) um einen LOHC-Träger handelt, liegt der Träger zur Aufnahme von Wasserstoff in seiner vollständig oder zumindest teilweise dehydrierten Form vor. Vorzugsweise handelt es sich dabei um die vollständig dehydrierte Form. Eine zumindest teilweise dehydrierte Form kann dann vorliegen, wenn beispielsweise eine vollständig dehydrierte Form eines solchen Trägers über zwei oder mehrere Stellen verfügt, an denen das entsprechende Molekül hydriert werden kann, und mindestens eine dieser zur Hydrierung geeigneten Stellen bereits in hydrierter Form vorliegt, aber noch mindestens eine Stelle in dehydrierter (nicht hydrierter) Form vorliegt. Beispiele hierfür sind unter anderem aromatische Verbindungen, insbesondere polyzyklische aromatische Verbindungen mit einem konjugierten π-Elektronensystem.

Das Inkontaktbringen des Trägers (T1) mit Wasserstoff kann prinzipiell nach beliebigen, dem Fachmann bekannten Methoden durchgeführt werden, beispielsweise indem Wasserstoff über den Träger (T1) übergeleitet wird, durch den Träger (T1) durchgeleitet wird oder auf den Träger (T1) aufgepresst wird.

Durch das Inkontaktbringen des Trägers (T1) mit Wasserstoff wird, wie es beispielsweise im nachfolgend definierten optionalen Schritt d) des erfindungsgemäßen Verfahrens durchgeführt wird, somit ein mit Wasserstoff beladener Träger (T2) erhalten. Sowohl der Träger (T1) als auch der durch Inkontaktbringen mit Wasserstoff erhaltene mit Wasserstoff beladene Träger (T2) können als Gemisch von zwei oder mehreren Trägern (T1) und/oder (T2) vorliegen. Sofern der Wasserstoff chemisch an den Träger (T1) gebunden wird, handelt es sich dabei um eine vollständige oder zumindest teilweise Hydrierung des entsprechenden Trägers (T1), sodass sich der mit Wasserstoff beladene Träger (T2) chemisch vom entsprechenden unbeladenen Träger (T1) unterscheidet. Vorzugsweise handelt es sich dabei um eine vollständige Hydrierung des Trägers (T1) mit Wasserstoff unter Erhalt des mit Wasserstoff beladenen Trägers (T2).

Das Verhältnis der Träger (T1) und (T2) im erfindungsgemäßen Verfahren gemäß Schritt a) wird nachfolgend an dem aus dem Stand der Technik bekannten LOHC-Träger Toluol erläutert. Toluol enthält einen Benzolring und somit drei π-Elektronenpaare. Toluol stellt also einen Träger (T1) dar, der in seiner vollständig dehydrierten Form vorliegt. Durch Inkontaktbringen des Toluols mit Wasserstoff wird erfindungsgemäß eine zumindest teilweise oder vorzugsweise vollständige Hydrierung von Toluol zu Methylcyclohexan als Träger (T2) erzielt. Sofern die vollständige Hydrierung von Toluol durchgeführt wird, werden also alle drei π-Elektronenpaare des Toluols hydriert, sodass Methylcyclohexan die vollständig hydrierte Form des Toluols darstellt. Sofern nur ein oder zwei der π-Elektronenpaare des Toluols hydriert worden sind, handelt es sich nur um eine teilweise Hydrierung. Solche zumindest teilweise hydrierten Verbindungen können sowohl als Träger (T1) oder als Träger (T2) erfindungsgemäß eingesetzt werden.

Normalerweise ist der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol.

Bei Verbindungen mit mindestens einer Kohlenstoff-Kohlenstoff-Doppelbindung (C-C-Doppelbindung) kann es sich beispielsweise um ein Olefin, einen Aromaten oder einen Heteroaromaten handeln. Solche Verbindungen sind dem Fachmann prinzipiell bekannt. Diese Verbindungen können gegebenenfalls substituiert sein, beispielsweise durch eine oder mehrere Alkylgruppen von beliebiger Kettenlänge, wie Methyl, Ethyl, Propyl oder durch sonstige funktionelle Gruppen, wie Halogen, Amino oder Carboxyl. Heteroaromatische Verbindungen weisen als Heteroatom mindestens ein Heteroatom auf, das vorzugsweise Sauerstoff, Stickstoff und/oder Schwefel ist. Aromatische oder heteroaromatische Verbindungen können monozyklisch, bizyklisch oder gegebenenfalls polyzyklisch sein. Gegebenenfalls können die entsprechenden Zyklen (Ringe) zumindest teilweise auch in hydrierter Form vorliegen. Ebenso ist es denkbar, dass beispielsweise bei einer bizyklischen Verbindung einer der beiden Zyklen vollständig hydriert ist.

Sofern als Träger (T1) ein N-Alkylcarbazol eingesetzt wird, kann der an das Stickstoffatom gebundene Alkylrest eine beliebige Kettenlänge annehmen, beispielsweise kann es sich dabei um Methyl, Ethyl, Propyl, Isopropyl oder Butyl sowie gegebenenfalls Mischungen davon handeln. Vorzugsweise handelt es sich dabei um N-Ethylcarbazol.

Demzufolge wird im erfindungsgemäßen Verfahren in Schritt a) vorzugsweise die entsprechende teilweise oder insbesondere vollständig hydrierte Form der vorstehend definierten konkreten Träger (T1) als mit Wasserstoff beladener Träger (T2) eingesetzt.

Erfindungsgemäß ist es bevorzugt, dass vor Durchführung von Schritt a) der mit Wasserstoff beladene Träger (T2) in einer (weiter unten genauer definierten) Vorrichtung (V4) gelagert wird, vorzugsweise ist die Vorrichtung (V4) ausgewählt aus einem Tank, einen Fermenter oder einem Behältnis, das durch Umrüsten einer Biogasanlage, insbesondere durch Umrüstung eines Fermenters, erhalten wurde oder das Bestandteil einer Biogasanlage ist. Weiterhin ist es bevorzugt, dass der mit Wasserstoff beladene Träger (T2) gemäß dem nachfolgend definierten optionalen Schritt d) hergestellt wird.

Schritt a) kann in jeder, dem Fachmann bekannten Vorrichtung (V1) durchgeführt werden, die insbesondere ein chemischer Reaktor ist. Wie weiter unten aufgeführt, ist es möglich, dass der optionale Verfahrensschritt d) ebenfalls in der Vorrichtung (V1) und/oder in der Vorrichtung (V4) durchgeführt wird. Vorzugsweise wird der Verfahrensschritt a) in einer separaten Vorrichtung (V1) durchgeführt. Die erfindungsgemäßen Verfahrensschritte a) und d) sowie die gegebenenfalls zwischengeschaltete Lagerung des mit Wasserstoff beladenen Trägers (T2) und/oder des Trägers (T1) werden also vorzugsweise jeweils in separaten Vorrichtungen durchgeführt. Prinzipiell ist es möglich, dass die Vorrichtung (V1) zur Durchführung des erfindungsgemäßen Schritts a) in einer von der Art her gleichen Vorrichtung durchgeführt wird wie die nachfolgend beschriebene Vorrichtung (V6) zur Durchführung des optionalen Verfahrensschritts d). Geeignete Vorrichtungen zur Durchführung des erfindungsgemäßen Verfahrensschritts a) sind beispielsweise in DE-B 10 2013 223 589 offenbart.

Das Freisetzen des Wasserstoffs gemäß Verfahrensschritt a) erfolgt vorzugsweise in einem druckstabilen chemischen Reaktor bei einer Prozesstemperatur zwischen 100°C und 450°C, bevorzugt zwischen 150°C und 420°C und insbesondere zwischen 180°C und 390°C. Der Prozessdruck liegt zwischen 0,1 und 30 bar, insbesondere zwischen 1 und 10 bar, wobei insbesondere ein metallhaltiger Katalysator eingesetzt werden kann, der insbesondere Platin und/oder Palladium enthält. Wesentlich ist, dass der Katalysator geeignet ist, Wasserstoff, der vorzugsweise von einem LOHC-Träger (T2) abgegeben wird, als Wasserstoffgas freizusetzen. Neben Platin und/oder Palladium sind dafür insbesondere Metalle, wie Chrom, Eisen, Kobalt, Nickel, Kupfer, Iridium oder Ruthenium, geeignet.

Weiterhin ist es bevorzugt, dass bei der Freisetzung von Wasserstoff gemäß Schritt a) zusätzlich der Träger (T1) wiedergewonnen wird.

Weiterhin ist es bevorzugt, dass bei der Freisetzung von Wasserstoff gemäß Schritt a) zusätzlich der Träger (T1) wiedergewonnen wird und der Träger (T1) zur Durchführung des weiter unten definierten optionalen Schritts d) verwendet wird.

Weiterhin ist es bevorzugt, dass bei der Freisetzung von Wasserstoff gemäß Schritt a) zusätzlich der Träger (T1) wiedergewonnen wird und anschließend der Träger (T1) in einer separaten Vorrichtung (V5) oder in der Vorrichtung (V4) gelagert wird. Im Anschluss an eine Lagerung, beispielsweise in der Vorrichtung (V5), wird der Träger (T1) vorzugsweise zur Durchführung des weiter unten definierten optionalen Schritts d) verwendet. Die Vorrichtungen (V4) bzw. (V5) als solche werden weiter unten genauer definiert.

Vorstehend wurde bereits erwähnt, dass die Wasserstofffreisetzung gemäß Schritt a) ein endothermer Prozess ist, also das zur Freisetzung des Wasserstoffs aus dem (mit Wasserstoff beladenen) Träger (T2) die Einspeisung von Energie erforderlich ist. Erfindungsgemäß wird die Freisetzung von Wasserstoff aus dem mit Wasserstoff beladenen Träger (T2) gemäß Schritt a) so durchgeführt, dass zumindest eine Teilmenge der für die endotherme Wasserstofffreisetzung benötigten Energie in Form von Wärme bereitgestellt wird, die durch die Verbrennung von Klärschlamm gewonnen wird. Vorzugsweise stammt mindestens 50 %, mehr bevorzugt mindestens 75 %, der für die Wasserstofffreisetzung in Schritt a) benötigten Energie aus der Verbrennung von Klärschlamm. In einer Ausführungsform der vorliegenden Erfindung stammt die für die Wasserstofffreisetzung in Schritt a) benötigten Energie vollständig, oder zumindest weitgehend vollständig (also mindestens 95 %), aus der Verbrennung von Klärschlamm. Die Durchführung einer Klärschlammverbrennung bzw. die damit verbundene Energiefreisetzung wird weiter unten am Beispiel der im optionalen Schritt c) gewonnenen Wärme näher erläutert.

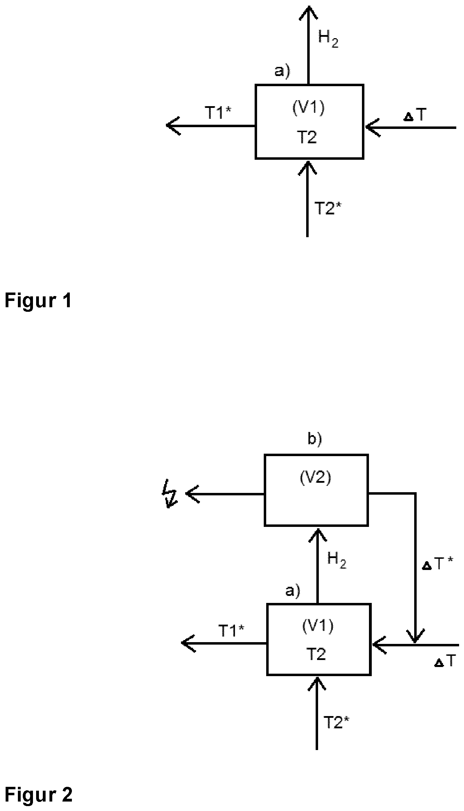

Figur 1 verdeutlicht das erfindungsgemäße Verfahren in seiner breitesten Form unter Berücksichtigung des Schritts a). Zunächst wird gemäß Schritt a) in der Vorrichtung (V1), die vorzugsweise ein chemischer Reaktor ist, Wasserstoff aus einem geeigneten, mit Wasserstoff beladenen Träger (T2), vorzugsweise ist der Träger (T2) in einem LOHC-Verfahren hergestellt worden, durch Energiezufuhr freigesetzt. Zumindest eine Teilmenge der zur Wasserstofffreisetzung erforderlichen Energie wird durch die Verbrennung von Klärschlamm gewonnen. Vorzugsweise stammt mindestens 50 %, mehr bevorzugt mindestens 75 %, der für die Wasserstofffreisetzung in Schritt a) benötigten Energie aus der Verbrennung von Klärschlamm.

Der mit Wasserstoff beladene Träger (T2) kann sich bereits in der Vorrichtung (V1) befinden, vorzugsweise wird der mit Wasserstoff beladene Träger (T2) in die Vorrichtung (V1) eingespeist, was in Figur 1 durch das Symbol (T2*) dargestellt ist. Der freigesetzte Wasserstoff und/oder der bei der Wasserstofffreisetzung gebildete Träger (T1) können in der Vorrichtung (V1) verbleiben. Vorzugsweise werden jedoch sowohl der freigesetzte Wasserstoff als auch der bei der Wasserstofffreisetzung gebildete Träger (T1) aus der Vorrichtung (V1), gegebenenfalls kontinuierlich, entfernt. Letzteres ist in Figur 1 bildlich dargestellt, was im Fall des bei der Wasserstofffreisetzung gebildeten Trägers (T1) durch das Symbol (T1*) dargestellt ist. Die einzelnen Schritte und/oder das gesamte Verfahren kann sowohl batchweise als auch kontinuierlich betrieben werden.

Erfindungsgemäß ist es bevorzugt, dass nach Schritt a) ein optionaler Verfahrensschritt b) durchgeführt wird, wobei in Schritt b) die Überführung des in Schritt a) erhaltenen Wasserstoffs in eine Vorrichtung (V2) erfolgt, wobei in der Vorrichtung (V2) Wasserstoff in elektrischen Strom und gegebenenfalls in Wärme umgewandelt wird.

Der in Schritt a) freigesetzte Wasserstoff kann zwar problemlos gelagert oder transportiert werden, vorzugsweise wird der optionale Schritt a) jedoch in räumlicher und/oder zeitlicher Nähe zu Schritt b) durchgeführt.

Die Vorrichtung (V2) als solche ist dem Fachmann bekannt. Vorzugsweise ist die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor.

Die vorgenannten Vorrichtungen (V2) sind häufig bereits in einer konventionellen Biogasanlage enthalten und können in aller Regel ohne größere Umrüstungen im erfindungsgemäßen Verfahren eingesetzt werden. Dies trifft insbesondere für ein Blockheizkraftwerk und/oder einen Gasmotor zu, die häufig in Biogasanlagen verwendet werden.

In dieser Ausführungsform ist es bevorzugt, dass sich die Vorrichtungen (V1) und (V2) in räumlicher Nähe zueinander befinden, beispielsweise auf demselben Betriebsgelände oder auf einem benachbarten Betriebsgelände. Ebenso werden die Schritte a) und b) vorzugsweise in zeitlicher Nähe zueinander, insbesondere parallel, durchgeführt.

Ebenso ist es bevorzugt, dass zumindest eine Teilmenge der für die Wasserstofffreisetzung in Schritt a) benötigten Energie in Form von Wärme bereitgestellt wird, die bei der Stromerzeugung gemäß Schritt b) zusätzlich gewonnen und die nach Schritt a) rückgeleitet wird. Die in den beiden Schritten a) und b) jeweils anfallenden Wärmemengen können entweder vollkommen unabhängig voneinander oder gemeinsam/zeitgleich als Energiequelle für die Freisetzung von Wasserstoff gemäß Schritt a) verwendet werden.

Figur 2 zeigt eine bevorzugte Ausführungsform des erfindungsgemäßen Verfahrens unter Berücksichtigung der Schritte a) und b). In Zusammenhang mit der Durchführung des erfindungsgemäßen Schritts a) in Figur 2 wird zunächst auf Figur 1 sowie die zugehörigen Ausführungen verwiesen, wo das erfindungsgemäße Verfahren in seiner breitesten verdeutlicht ist. Zusätzlich zu Figur 1 ist in der bevorzugten Ausführungsform gemäß Figur 2 mitberücksichtigt, dass der in Schritt a) erhaltene Wasserstoff in eine Vorrichtung (V2) überführt wird, wobei in der Vorrichtung (V2) Wasserstoff in elektrischen Strom und gegebenenfalls in Wärme umgewandelt wird. Die optionale Rückleitung der in Schritt b) erhaltenen Wärme zu Freisetzung von Wasserstoff gemäß Schritt a) ist in Figur 2 ebenfalls dargestellt.

Figur 2 verdeutlicht somit den zweiten Erfindungs-Gegenstand der vorliegenden Erfindung, das Verfahren zur Erzeugung von elektrischem Strom sowie gegebenenfalls von Wärme. Es ist dabei bevorzugt, dass sich die Vorrichtungen (V1) und (V2) in räumlicher Nähe zueinander befinden, beispielsweise auf demselben Betriebsgelände oder auf einem benachbarten Betriebsgelände. Ebenso werden die Schritte a) und b) vorzugsweise in zeitlicher Nähe zueinander, insbesondere parallel, durchgeführt.

Erfindungsgemäß ist es bevorzugt, dass vor Schritt b) ein Schritt c) durchgeführt wird:
c) Verbrennen des getrockneten Klärschlamms in einer Vorrichtung (V3).

Als Vorrichtung (V3) kommen prinzipiell alle dem Fachmann bekannten Vorrichtungen infrage, mit denen getrockneter Klärschlamm verbrannt werden kann. Geeignete Vorrichtungen (V3) werden auch als Klärschlammverbrennungsanlagen bezeichnet. Die Vorrichtung (V3) umfasst mindestens eine Verbrennungseinheit sowie gegebenenfalls weitere Einheiten beispielsweise zur Zuführung und/oder Abführung von Wärme, vorzugsweise Wärmetauscher oder Heizbündel. Gegebenenfalls können in der Vorrichtung (V3) auch zwei oder mehr Verbrennungseinheiten enthalten sein, die beispielsweise parallel oder in Reihe geschaltet betrieben werden, und/oder sonstige Behältnisse, beispielsweise ein oder mehrere Vorrat-Tanks zur Lagerung von Klärschlamm-Chargen in unterschiedlichen Trockenstufen. Insbesondere umfasst die Vorrichtung (V3) Elemente zur Steuerung des Abgases (Abgasstrom), der bei der Verbrennung des getrockneten Klärschlamms entsteht. Sinngemäßes gilt auch für die bei der Klärschlammverbrennung freigesetzte Wärme.

Erfindungsgemäß ist es bevorzugt, dass in Schritt c) bei der Klärschlammverbrennung Wärme freigesetzt wird und diese freigesetzte Wärme im Rahmen des erfindungsgemäßen Verfahrens weiter verwendet wird, wobei vorzugsweise
i) die Wärme aus dem Abgas der Verbrennungsanlage abgegeben wird, und/oder
ii) zumindest eine Teilmenge der freigesetzten Wärme, vorzugsweise unter Verwendung eines Heizbündels und/oder eines Wärmetauschers, nach Schritt a) überführt wird, um Wasserstoff aus dem Träger (T2) freizusetzen.

In einer ersten bevorzugten Ausführungsform der vorliegenden Erfindung umfasst das Verfahren zur Erzeugung von Wasserstoff die Schritte a), b) und c) gemäß den vorstehenden Definitionen.

Erfindungsgemäß ist es weiterhin bevorzugt, dass mit dem (in Schritt a)) bei der Freisetzung des Wasserstoffs erhaltenen Träger (T1) ein Schritt d) ausgeführt wird, wobei
d) Inkontaktbringen eines Trägers (T1) mit Wasserstoff zur Speicherung des Wasserstoffs unter Erhalt eines mit Wasserstoff beladenen Trägers (T2) sowie Freisetzung von Wärme.

Prinzipiell ist es möglich, dass der optionale Schritt d) in der gleichen Vorrichtung (V4) durchgeführt wird wie die vorstehend bereits erwähnte Lagerung des mit Wasserstoff beladenen Trägers (T2), die vor Durchführung des Schritts a) optional durchgeführt werden kann. Dies kann erfolgen, wenn beispielsweise in eine solche Vorrichtung (V4) eine spezielle Einheit/Vorrichtungsbestandteil integriert wird, der die Durchführung des Verfahrensschritts d) getrennt von der Durchführung der Lagerung des mit Wasserstoff beladenen Trägers (T2) ermöglicht. Entsprechende Einheiten oder Einbaumaßnahmen sind dem Fachmann bekannt. Vorzugsweise wird jedoch im erfindungsgemäßen Verfahren Schritt d) in einer separaten Vorrichtung (V6) durchgeführt, die vorzugsweise ein chemischer Reaktor ist. Weiterhin ist es bevorzugt, dass der in Schritt d) des erfindungsgemäßen Verfahrens gebildete mit Wasserstoff beladenen Träger (T2) nicht in der Vorrichtung (V6) verbleibt, sondern wie nachfolgend ausgeführt zur Lagerung in eine separate Vorrichtung, beispielsweise in die Vorrichtung (V4) überführt wird.

Vorzugsweise wird Schritt d) in einer druckstabilen Vorrichtung, insbesondere in einem druckstabilen chemischen Reaktor, durchgeführt. Weiterhin ist es bevorzugt, dass Schritt d) bei einer Temperatur zwischen 50°C und 400°C, insbesondere zwischen 120°C und 300°C, insbesondere zwischen 150°C und 280°C durchgeführt wird. Die Hydrierung, also das Beladen des Trägers (T1) mit Wasserstoff, findet vorzugsweise bei einem Verfahrensdruck von 2 bar bis 200 bar, insbesondere bei 10 bar bis 100 bar, statt. Weiterhin ist es bevorzugt, dass Schritt d) in Gegenwart eines metallhaltigen Katalysators durchgeführt wird. Als Katalysatoren für die Beladung des LOHC-Trägers (T1) eignen sich insbesondere solche, die das Element Ruthenium und/oder Nickel aufweisen. Es sind auch Katalysatoren möglich, die andere Elemente oder zusätzliche Elemente neben Ruthenium und/oder Nickel aufweisen. Wesentlich sind solche Elemente, die Wasserstoff anlagern und auf LOHC-Träger (T1) übertragen können. Neben Ruthenium und/oder Nickel sind insbesondere Metalle wie Chrom, Eisen, Kobalt, Kupfer, Iridium, Palladium oder Platin als Katalysatoren möglich.

Vorzugsweise wird der erfindungsgemäße Schritt d) räumlich getrennt und/oder zeitlich getrennt von den vorstehend beschriebenen Schritten a) und/oder b) durchgeführt, vorzugsweise in einer Entfernung von mindestens 5 km, insbesondere 10 bis 50 km, und/oder Schritt d) wird mindestens einen Monat, besonders bevorzugt mindestens drei Monate, nach den vorstehend beschriebenen Schritten a) und/oder b) durchgeführt. Gegebenenfalls kann der Schritt d) aber auch in räumlicher Nähe und/oder in direkter zeitlicher Abfolge zu Schritt a) durchgeführt werden.

Wie vorstehend bereits erwähnt wird im erfindungsgemäßen Schritt d) zusätzlich Wärme freigesetzt aufgrund der exothermen Speicherung des Wasserstoffs durch Inkontaktbringen mit dem Träger (T1). Erfindungsgemäß ist es bevorzugt, dass die in Schritt d) freigesetzte Wärme im erfindungsgemäßen Verfahren wiederverwendet wird.

Erfindungsgemäß ist es somit ebenfalls bevorzugt dass nach Schritt d) ein Schritt e) durchgeführt wird, wobei
e) Einspeisung der in Schritt d) freigesetzten Wärme in eine Vorrichtung (V7), um in der Vorrichtung (V7) Klärschlamm zu trocknen.

Die Durchführung einer Klärschlammtrocknung als solcher ist einem Fachmann bekannt. Sinngemäßes gilt auch für die (chemische) Beschaffenheit von Klärschlamm als solchen, geeigneten Vorrichtungen zur Durchführung der Klärschlammtrocknung und/oder für die Aufarbeitung/Weiterverwendung von getrocknetem Klärschlamm, beispielsweise durch Verbrennen von Klärschlamm. Hintergrundinformationen in diesem Kontext können beispielsweise den eingangs erwähnten Artikeln "Klärschlamm-Entsorgung in der Bundesrepublik Deutschland" (Stand: Oktober 2018, herausgegeben vom Bundesumweltamt, erhältlich über das Internet unter: www.umweltbundesamt.de/publikationen) oder "Klärschlammtrocknung in Deutschland - Stand und Perspektiven" von Jürgen Geyer (Seiten 927-948; in Energie aus Abfall - Band 10; TK Verlag Karl Thomé-Kozmiensky; Neuruppin 2013) entnommen werden.

Als Vorrichtung (V7) kommen prinzipiell alle dem Fachmann bekannten Vorrichtungen infrage, mit denen Klärschlamm durch Wärmezufuhr getrocknet werden kann. Die Vorrichtung (V7) umfasst mindestens einen Klärschlammtrockner. Gegebenenfalls können in der Vorrichtung (V7) auch zwei oder mehr Klärschlammtrockner enthalten sein, die beispielsweise parallel oder in Reihe geschaltet betrieben werden, und/oder sonstige Behältnisse, beispielsweise ein oder mehrere Vorrat-Tanks zur Lagerung von Klärschlamm-Chargen in unterschiedlichen Trockenstufen. Weiterhin kann die Vorrichtung (V7) auch an einen oder mehrere Wärmetauscher angeschlossen sein bzw. solche Wärmetauscher können in die Vorrichtung (V7) integriert sein.

Vorzugsweise ist die Vorrichtung (V7) mindestens ein Klärschlammtrockner ausgewählt aus einem Bandtrockner, einem Trommeltrockner oder einem Scheibentrockner. Solche Klärschlammtrockner sind kommerziell erhältlich, beispielsweise unter der Bezeichnung "Huber Bandtrockner BT" der Firma Huber SE (Berching, Deutschland).

Die in Schritt d) freigesetzte Wärme kann beispielsweise über eine Rohrleitung oder ein Rohrleitungssystem in die Vorrichtung (V7) geleitet werden. Um den Wärmetransport von Schritt d) nach Schritt e) möglichst effizient zu gestalten, ist es vorteilhaft, wenn dabei Wärmetauscher eingesetzt werden.

Im Rahmen der vorliegenden Erfindung ist es somit bevorzugt, dass in Schritt e)
i) die Vorrichtung (V7) mindestens einen Klärschlammtrockner umfasst ausgewählt aus einem Bandtrockner, einem Trommeltrockner oder einem Scheibentrockner, und/oder
ii) die in Schritt d) freigesetzte Wärme über eine Rohrleitung oder ein Rohrleitungssystem in die Vorrichtung (V7) geleitet wird, und/oder
iii) die in Schritt d) freigesetzte Wärme durch einen Wärmetauscher geleitet wird, um die Trocknungsluft in der Vorrichtung (V7), insbesondere in einem Bandtrockner, aufzuheizen, und/oder
iv) die Vorrichtung (V7) mindestens zwei Bereiche mit unterschiedlichen Temperaturzonen aufweist.

Aufgrund des im erfindungsgemäßen Verfahren vorzugsweise stattfindenden Wärmetransports von Schritt d) und Schritt e) ist es vorteilhaft, wenn die beiden Schritte in räumlicher Nähe voneinander durchgeführt werden, also die jeweiligen Vorrichtungen zur Durchführung dieser Schritte vorzugsweise nicht weit voneinander entfernt aufgestellt sind. Abstände von beispielsweise 500 m bis 1 km zwischen den jeweiligen Vorrichtungen zur Durchführung der beiden Schritte d) und e) sind in der Praxis problemlos zu bewältigen.

Zwar ist es denkbar bzw. technisch realisierbar, dass sich die jeweiligen Vorrichtungen zur Durchführung der beiden Schritte auch im einen größeren räumlichen Abstand zueinander finden, jedoch ist der Energieverlust umso größer, je weiter dieser Abstand zwischen den jeweiligen Vorrichtungen zur Durchführung der beiden Schritte d) und e) ist. Durch entsprechend ausgestaltete Rohrleitungen, beispielsweise spezielle isolierte Rohrleitungen, können auch größere Abstände überwunden werden, ohne dass es zu einem signifikanten Wärmeverlust kommen muss. Dies ist aber insofern mit einem Nachteil verbunden, weil dadurch die Installationskosten wieder ansteigen. Denkbar ist, dass sich die jeweiligen Vorrichtungen auf unterschiedlichen Betriebsgeländen in näherer räumlicher Umgebung befinden.

Im Rahmen der vorliegenden Erfindung ist es weiterhin bevorzugt, dass die beiden Schritte d) und e) in zeitlicher Nähe, beispielsweise parallel zueinander, durchgeführt werden. Technisch ist es auch möglich, dass die freigesetzte Wärme zwischengelagert wird und die beiden Schritte zeitlich versetzt zueinander durchgeführt werden.

Vorteilhaft bei der Durchführung des erfindungsgemäßen Schritts e) ist es weiterhin, wenn die Einspeisung der in Schritt d) freigesetzten Wärme in die Vorrichtung (V7) so erfolgt, dass die Vorrichtung (V7) mindestens zwei Bereiche mit unterschiedlichen Temperaturzonen aufweist. Die Vorrichtung (V7) kann dabei zwei oder mehrere Temperaturzonen aufweisen, die beispielsweise dadurch gesteuert werden, dass die Wärme an unterschiedlichen Stellen und/oder in unterschiedlichen Mengen und/oder durch Verwendung von Wärmetauschern in die Vorrichtung (V7) eingespeist wird. Beispielsweise kann die Vorrichtung (V7) mindestens eine Niedertemperaturzone und/oder mindestens eine Hochtemperaturzone umfassen. Die entsprechenden Temperaturbereiche, die zur Trocknung von Klärschlamm in der Vorrichtung (V7) in den jeweiligen Zonen oder Bereiche der entsprechenden Vorrichtung eingestellt werden können, sind dem Fachmann bekannt.

Bevorzugt ist es, dass die Vorrichtung (V7) a) eine Niedertemperaturzone (Z1) umfasst, die bei einem Temperaturniveau von 50 bis 90 °C, vorzugsweise bei 60 bis 80 °C, insbesondere bei 70 °C, betrieben wird, und b) eine Hochtemperaturzone (Z2) umfasst, die bei einem Temperaturniveau von 100 bis 180 °C, vorzugsweise bei 130 bis 150 °C, insbesondere bei 140 °C, betrieben wird.

Durch entsprechende Regulierung der Wärmeeinspeisung in die Vorrichtung (V7) und/oder die Verweildauer des Klärschlamms in der Vorrichtung (V7) kann der Fachmann den Trocknungsgrad des Klärschlamms in Schritt e) steuern. Ebenso weiß der Fachmann, dass der Wassergehalt des zu trocknenden Klärschlamms und/oder dessen chemische Zusammensetzung die Trocknungsdauer und/oder den Trocknungsgrad des Klärschlamms in Schritt e) beeinflussen können. Der Fachmann weiß aufgrund seines Fachwissens und/oder gegebenenfalls unter Zuhilfenahme der vorstehend zu Beginn der Ausführungen zu Schritt e) aufgeführten Literaturstellen, wie für die unterschiedlichen Arten von Klärschlamm der gewünschte bzw. ein hoher Trocknungsgrad eingestellt werden kann.

Sofern nichts anderes angegeben, wird die Trocknung des Klärschlamms im Rahmen der vorliegenden Erfindung in Prozent angegeben und bezieht sich auf den Trockenrückstand (TR). Dieser ist als Maß für den Gehalt an Feststoff der nicht abfiltrierten Schlammprobe bzw. den Anteil der Trockenmasse an der gesamten Schlammmasse definiert. Die Bestimmung erfolgt durch Verdampfung des Wassers (siehe auch Tab. 1 auf Seite 7 des vorstehend erwähnten Artikels "Klärschlamm-Entsorgung in der Bundesrepublik Deutschland").

Vorzugsweise wird in Schritt e) getrockneter Klärschlamm erhalten, der in der Vorrichtung (V7) bis zu einem Trocknungsgrad von mindestens 75 %, vorzugsweise bei mindestens 85 %, insbesondere bei 95 bis 100 % (Volltrocknung) getrocknet wird.

Im Rahmen der vorliegenden Erfindung ist es somit weiterhin bevorzugt, dass
i) Schritt d) und Schritt e) in räumlicher Nähe durchgeführt werden, vorzugsweise befinden sich die jeweiligen Vorrichtungen zur Durchführung der beiden Schritte d) und e) auf demselben Betriebsgelände, und/oder
ii) die Vorrichtung (V7) umfasst a) eine Niedertemperaturzone (Z1), die bei einem Temperaturniveau von 50 bis 90 °C, vorzugsweise bei 60 bis 80 °C, insbesondere bei 70 °C, betrieben wird, und b) eine Hochtemperaturzone (Z2), die bei einem Temperaturniveau von 100 bis 180 °C, vorzugsweise bei 130 bis 150 °C, insbesondere bei 140 °C, betrieben wird, und/oder
iii) der Klärschlamm in der Vorrichtung (V7) bis zu einem Trocknungsgrad von mindestens 75 %, vorzugsweise bei mindestens 85 %, insbesondere bei 95 bis 100 % (Volltrocknung) getrocknet wird.

Wie bereits erwähnt, kann der in der Vorrichtung (V7) zu trocknende Klärschlamm über eine beliebige, dem Fachmann bekannte chemische Zusammensetzung verfügen und/oder beliebige Trocknungsgrade aufweisen. Der zu trocknende Klärschlamm wird auch als nasser Klärschlamm oder gegebenenfalls auch als Dünnschlamm bezeichnet. Beispielsweise kann im Rahmen der Erfindung der nasse Klärschlamm zunächst einer Vortrocknung unterzogen worden sein, um zunächst den Wassergehalt in einer Vorstufe zu reduzieren. Dies wird auch als Entwässerung von Klärschlamm bezeichnet, wodurch ein (nasser) Klärschlamm mit einem Trocknungsgrad von 20 bis 30 % erhalten werden. Diese Entwässerung des Klärschlamms in einer Vorstufe kann beispielsweise mit einer Schneckenpresse erfolgen, die beispielsweise kommerziell erhältlich ist unter der Bezeichnung "Huber Schneckenpresse Q-Press" der Firma Huber SE (Berching, Deutschland).

Weiterhin kann der nasse Klärschlamm zunächst in einer separaten Vorrichtung zwischengelagert werden bevor er in die Vorrichtung (V7) zur Durchführung des erfindungsgemäßen Schritt e) eingespeist wird. Ebenso ist es denkbar, dass nasser Klärschlamm beispielsweise von einem Lkw angeliefert wird und direkt an die Vorrichtung (V7) eingespeist wird. Die Zwischenlagerung des nassen Klärschlamms kann beispielsweise auch in einem Fermenter, einem Nachgärer oder einer Gärresteanlage erfolgen. Die Lagerung und/oder der Transport und/oder die Überführung von nassem Klärschlamm ist einem Fachmann bekannt, beispielsweise kann durch Zuhilfenahme von speziellen Pumpen nasser Klärschlamm in die Vorrichtung (V7) aus einem entsprechenden Vorratsbehälter eingespeist werden.

Im Rahmen der vorliegenden Erfindung ist es somit weiterhin bevorzugt, dass
i) der Klärschlamm zunächst entwässert wird und mit einem Trocknungsgrad von 20 bis 30 % vorliegt, bevor er in die Vorrichtung (V7) zur Trocknung eingespeist wird, und/oder
ii) der Klärschlamm mit einer Dickstoffpumpe in die Vorrichtung (V7) zur Trocknung eingespeist wird, und/oder
iii) der Klärschlamm vor der Trocknung in der Vorrichtung (V7) in einer Vorrichtung (V8) gelagert wird, vorzugsweise ist die Vorrichtung (V8) ausgewählt aus einem Tank, einem Fermenter oder einem Behältnis, das durch Umrüsten einer Biogasanlage, insbesondere durch Umrüstung eines Fermenters, erhalten wurde oder das Bestandteil einer Biogasanlage ist.

Erfindungsgemäß ist es weiterhin bevorzugt, dass der in Schritt e) getrocknete Klärschlamm in die Vorrichtung (V3) überführt wird, um gemäß (dem vorstehend bereits definierten) Schritt c) getrockneten Klärschlamm in der Vorrichtung (V3) zu verbrennen.

Die erforderlichen Vorrichtungen (V7) zur Trocknung des Klärschlamms gemäß Schritt e) sowie (V3) zur Verbrennung des getrockneten Klärschlamms gemäß Schritt c) können in der Praxis sich in räumlicher Nähe zueinander befinden, beispielsweise auf demselben Betriebsgelände, vorzugsweise befinden sich die jeweiligen Vorrichtungen jedoch in einem größeren räumlichen Abstand zueinander. Ebenso ist es bevorzugt, dass die Durchführung der beiden Schritte e) und c) zeitlich versetzt bzw. getrennt erfolgt. Ein Transport des getrockneten Klärschlamms (tKS) ist problemlos über beliebig viele Kilometer möglich, beispielsweise durch LKWs, Züge und/oder Schiffe. Eine Zwischenlagerung des getrockneten Klärschlamms (tKS) kann ebenfalls problemlos durchgeführt werden, beispielsweise in räumlicher Nähe zur Vorrichtung (V7) und/oder zur Vorrichtung (V3) bzw. entsprechende Zwischenlager können direkt in die jeweilige Vorrichtungen integriert sein. Ebenso ist es möglich, dass im Rahmen der vorliegenden Erfindung der getrocknete Klärschlamm (tKS) räumlich in großer Distanz zu den beiden Vorrichtungen (V7) und (V3) in einem geeigneten Zwischenlager, beispielsweise in einem Tank, untergebracht wird, bevor Schritt c) durchgeführt wird.

Im Rahmen der vorliegenden Erfindung ist es weiterhin bevorzugt, dass
i) vor Durchführung von Schritt c) der getrocknete Klärschlamm zwischengelagert wird, und/oder
ii) Schritt e) und Schritt c) räumlich getrennt voneinander durchgeführt werden, vorzugsweise in einer Entfernung von mindestens 5 km, insbesondere 10 bis 50 km, und/oder
iii) Schritt e) und Schritt c) zeitlich getrennt voneinander durchgeführt werden, vorzugsweise wird Schritt c) mindestens einen Monat, besonders bevorzugt mindestens drei Monate, nach Schritt e) durchgeführt.

In einer bevorzugten Ausführungsform der vorliegenden Erfindung wird in Anschluss an die Durchführung des optionalen Schritts d) sowie zeitlich unabhängig von der Durchführung des optionalen Schritts e) eine Lagerung des in Schritt d) erhaltenen, mit Wasserstoff beladenen Trägers (T2) durchgeführt. Die Lagerung des mit Wasserstoff beladenen Trägers (T2) kann dabei prinzipiell in der gleichen Vorrichtung durchgeführt werden, in der auch Schritt d) durchgeführt wird, beispielsweise in der Vorrichtung (V6). Vorstehend wurde bereits darauf hingewiesen, dass es im Rahmen der vorliegenden Erfindung bevorzugt ist, dass die Durchführung des Schritts d) sowie die nachfolgende Lagerung des dabei erhaltenen, mit Wasserstoff beladenen Trägers (T2) in unterschiedlichen Vorrichtungen durchgeführt werden.

Die Lagerung des mit Wasserstoff beladenen Trägers (T2) kann prinzipiell für beliebige Zeiträume stattfinden. In der Regel erfolgt die Lagerung für mindestens mehrere Tage, sie kann aber auch mehrere Wochen, Monate oder gar Jahre betragen. Genauso ist es aber auch denkbar, dass die Lagerung nur für wenige Minuten oder Stunden oder für einen Tag durchgeführt wird. Vorzugsweise erfolgt die Lagerung für mindestens zwei Tage und/oder maximal sechs Monate.

Ebenso ist es möglich, dass der mit Wasserstoff beladene Träger (T2) im Anschluss an seine Herstellung gemäß Schritt d) gar nicht in einer separaten Vorrichtung gelagert wird, sondern direkt weiterverarbeitet wird, indem er vorzugsweise zur geeigneten Vorrichtung (V1) zur Durchführung des erfindungsgemäßen Schritts a) transportiert wird, wo der Wasserstoff aus dem Träger (T2) wieder freigesetzt wird. Vorzugsweise werden Schritt a) und Schritt d) in räumlicher Distanz zueinander durchgeführt, noch mehr bevorzugt zusätzlich mit einem zeitlichen Abstand, sodass vorzugsweise eine separate Lagerung des mit Wasserstoff beladenen Trägers im Anschluss an die Durchführung von Schritt d) erfolgt.

Vorzugsweise wird diese Ausführungsform so durchgeführt, dass der in Schritt d) erhaltene, mit Wasserstoff beladene Träger (T2) in der Vorrichtung (V4) gelagert wird, vorzugsweise ist die Vorrichtung (V4) ausgewählt aus einem Tank, einen Fermenter oder einem Behältnis, das durch Umrüsten einer Biogasanlage, insbesondere durch Umrüstung eines Fermenters, erhalten wurde oder das Bestandteil einer Biogasanlage ist.

Als Vorrichtung (V4) kommen prinzipiell alle dem Fachmann bekannten Vorrichtungen in Frage, bevorzugt sind dabei solche Vorrichtungen, die Bestandteil einer Biogasanlage sind oder waren. Sofern eine Biogasanlage umgerüstet wird, müssen sich die entsprechenden Vorrichtungen (V4) zur Lagerung eines solchen mit Wasserstoff beladenen Trägers (T2) eignen und/oder sie müssen durch, vorzugsweise einfache, Schritte so umgerüstet werden, dass eine entsprechende Lagerung durchgeführt werden kann. Prinzipiell ist es denkbar, dass eine hierfür geeignete Vorrichtung (V4) kommerziell erworben wird und in eine bestehende Biogasanlage eingebaut wird. In anderen Worten ausgedrückt, kann bei dieser Ausführungsform eine neue Vorrichtung (V4) verwendet werden, die vorher noch nicht in einer Biogasanlage eingesetzt gewesen ist und/oder sich für die Verwendung zur Erzeugung von Biogas nicht eignet. Dies kann erfindungsgemäß insbesondere dann durchgeführt werden, wenn gemäß einer weiter unten beschriebenen bevorzugten Ausführungsform die entsprechende Biogasanlage weiterhin, beispielsweise zeitgleich (parallel) oder zeitversetzt, zur Herstellung von Biogas eingesetzt werden soll. Sofern jedoch eine entsprechende Biogasanlage nicht mehr zur Erzeugung von Biogas eingesetzt werden soll, wird erfindungsgemäß als Vorrichtung (V4) eine Vorrichtung verwendet, die bereits in der entsprechenden Biogasanlage verwendet worden ist. Die letzte Option ist selbstverständlich auch dann denkbar, wenn die entsprechende Anlage weiterhin zur Erzeugung von Biogas verwendet werden soll.

Biogasanlagen als solche sind, wie eingangs bereits erwähnt, dem Fachmann bekannt. In der Regel weisen Biogasanlagen (unter anderem) ein oder mehrere Fermenter, Vorrats- oder Lagerbehälter, Vorrichtungen zur Erzeugung von Strom, insbesondere Gasmotoren, sowie sonstige Leitungen auf. Wie vorstehend bereits erwähnt, kommen als Vorrichtung (V4) prinzipiell alle dem Fachmann bekannten Vorrichtungen in Frage, die Bestandteil einer Biogasanlage sind oder waren. Vorzugsweise handelt es sich dabei um einen Fermenter oder Nachgärer oder um Vorrichtungen, die durch Umrüstung aus einem Fermenter oder Nachgärer hergestellt worden sind. Erfindungsgemäß ist es mehr bevorzugt, dass die Vorrichtung (V4) ein Fermenter ist oder durch Umrüstung aus einem Fermenter hergestellt worden ist. Weiterhin ist es erfindungsgemäß bevorzugt, dass die Vorrichtung (V4) eine separate Vorrichtung ist gegenüber der Vorrichtung (V1), in der der erfindungsgemäße Verfahrensschritt a) durchgeführt wird.

Prinzipiell ist es denkbar, dass die Vorrichtung (V4) weiterhin als Fermenter genutzt wird. Bei dieser Fallkonstellation werden in die Vorrichtung (V4) entsprechende zusätzliche Vorrichtungsbestandteile oder Elemente eingebaut, die eine Lagerung des mit Wasserstoff beladenen Trägers (T2) in der entsprechenden Vorrichtung (V4), insbesondere in einem Fermenter, ermöglichen, ohne dass dabei der bisherige Verwendungszweck im Zusammenhang mit der Herstellung von Biogas eingestellt werden muss. Unter Umständen kann es dabei erforderlich sein, dass weitere Zuleitungen und/oder Ableitungen in die entsprechende Vorrichtung (V4), insbesondere in einen Fermenter, eingebaut werden müssen, um das Einbringen des Trägers (T2) sowie die im Anschluss an die Lagerung erforderliche Entnahme des Trägers (T2) aus der entsprechenden Vorrichtung, insbesondere dem Fermenter, zu ermöglichen.

Erfindungsgemäß ist es jedoch bevorzugt, dass die Vorrichtung (V4), insbesondere ein Fermenter, so umgebaut/umgerüstet wird, dass der bisherige Verwendungszweck im Rahmen der Biogasanlage nicht mehr durchgeführt werden kann. Im Falle eines Fermenters können beispielsweise aus der Vorrichtung (V4) die in einem Fermenter in der Regel vorhandenen Rührwerkzeuge und die damit verbundenen Bedienelemente oder Leitungen ausgebaut werden. Gegebenenfalls vorhandene Heizelemente können hingegen in der entsprechenden Vorrichtung (V4) ohne Probleme belassen werden, da sie auch im Zusammenhang mit der Lagerung des mit Wasserstoff beladenen Trägers (T2) sinnvoll verwendet werden können. Gegebenenfalls können die entsprechenden bisher vorhandenen Zuführ- und/oder Entnahmeelemente in der entsprechenden Vorrichtung (V4) zu den für das erfindungsgemäße Verfahren erforderlichen Zwecken umgerüstet werden oder gegebenenfalls ohne Umrüstung eingesetzt werden.

Vorzugsweise weist die Vorrichtung (V4) zwei räumlich getrennte Bereiche auf, wobei ein erster Bereich zur Lagerung des mit Wasserstoff beladenen Trägers (T2) verwendet wird und ein zweiter Bereich zur Lagerung des Trägers (T1) verwendet wird.

Weiterhin ist es bevorzugt, dass die Vorrichtung (V4) in ihrem Inneren mit einer Schutzauskleidung versehen wird, bevor die Lagerung des Trägers (T1) durchgeführt wird. Solche Schutzauskleidungen sind beispielsweise in EP-A 3 415 609 im Rahmen des dortigen optionalen Verfahrensschrittes g) beschrieben.

Weiterhin ist es bevorzugt, dass in die Vorrichtung (V4) eine schwimmende Trennwand eingebaut ist, wodurch der Träger (T1) getrennt von dem mit Wasserstoff beladenen Träger (T2) gelagert wird. Beispielsweise kann der Träger (T1) oberhalb und der Träger (T2) unterhalb der schwimmenden Trennwand gelagert werden.

Weiterhin ist es bevorzugt, dass die Vorrichtung (V4) Bestandteil einer Biogasanlage war und von allen Substanzen gereinigt war, die sich infolge der Erzeugung von Biogas in ihrem Inneren oder gegebenenfalls in ihren Zuleitungen befunden haben, bevor die Lagerung des Trägers (T2) durchgeführt wird. Des Weiteren kann die Vorrichtung (V4) auch einem zusätzlichen Reinigungsschritt unterzogen werden, wie er in EP-A 3 415 609 als dortiger optionaler Reinigungsschritt f) beschrieben ist.

Erfindungsgemäß ist es bevorzugt, dass die Vorrichtung (V4) sich in räumlicher Nähe zu der in Schritt d) eingesetzten Vorrichtung (V6) befindet, sofern für die Durchführung des Schritts d) und die Lagerung des dabei erhaltenen Trägers (T2) jeweils eine separate Vorrichtung verwendet wird, insbesondere befinden sich die entsprechenden Vorrichtungen auf demselben Betriebsgelände. Sinngemäßes gilt auch für eine etwaige separate Vorrichtung (V5), die wie vorstehend bereits ausgeführt für die Lagerung des in Schritt a) wiedergewonnenen Trägers (T1) verwendet werden kann.

In einer anderen Ausführungsform ist es bevorzugt, dass die Vorrichtung (V4) sich in räumlicher Nähe zu der im vorstehend bereits beschriebenen Schritt a) eingesetzten Vorrichtung (V1) befindet, insbesondere befinden sich die entsprechenden Vorrichtungen auf demselben Betriebsgelände. Sinngemäßes gilt auch für eine etwaige separate Vorrichtung (V5), die wie vorstehend bereits für die Lagerung des in Schritt a) wiedergewonnenen Trägers (T1) verwendet werden kann.

Die Vorrichtung (V4) dient vordergründig zur Lagerung des mit Wasserstoff beladenen Trägers (T2). Wie vorstehend bereits erwähnt ist es jedoch problemlos möglich, dass sowohl der Träger (T1) als auch der mit Wasserstoff beladenen Träger (T2) getrennt voneinander in der gleichen Vorrichtung, insbesondere einer Vorrichtung (V4) gelagert werden können. Sofern die Lagerung des Trägers (T1) in einer separaten Vorrichtung (V5) erfolgt, ist die Vorrichtung (V5) von ihrer Art her prinzipiell gleich wie die vorstehend beschriebene Vorrichtung (V4). Die Art und/oder Dauer der Lagerung des Trägers (T1) kann sinngemäß durchgeführt werden wie vorstehend für die Lagerung des mit Wasserstoff beladenen Trägers (T2) in der Vorrichtung (V4) beschrieben.

Im erfindungsgemäßen Verfahren ist es weiterhin bevorzugt, dass vor Schritt d) ein Schritt f) durchgeführt wird. Gemäß des optionalen erfindungsgemäßen Schritts f) erfolgt die Erzeugung von Wasserstoff in einer Vorrichtung (V9), vorzugsweise in einem Elektrolyseur, unter Verwendung von Wasser und elektrischem Strom. Die Durchführung des Verfahrensschritts f) als solchem ist dem Fachmann bekannt. Geeignete Elektolyseure sind beispielsweise in DE-B 10 2013 223 589 oder DE-A 10 2012 005 023 beschrieben. Hierbei kann es sich beispielsweise um einen sogenannten PEM-Elektrolyseur (Polymerelektrolytmembran) oder um einen SOEC-Elektrolyseur (solid oxide electolysis cell) sowie um einen Hochtemperatur-Elektrolyseur handeln. Letztendlich ist als Vorrichtung (V9) jede Art von Vorrichtung geeignet, in der die Zerlegung von Wasser in Wasserstoff oder Sauerstoff bei gleichzeitiger Einspeisung von elektrischem Strom durchgeführt werden kann. In der Vorrichtung (V9) sind in der Regel zwei Elektroden vorhanden. Bei dem eingesetzten Wasser handelt es sich normalerweise um VE-Wasser (vollentsalztes Wasser; siehe beispielsweise Siemens Silyzer-Broschüre).

Der in Schritt f) verwendete elektrische Strom kann prinzipiell aus beliebigen Quellen stammen. So ist es denkbar, dass der elektrische Strom einem separaten Netz (Verbrauchernetz) entnommen wird. Vorzugsweise handelt es sich jedoch bei dem eingesetzten Strom gemäß Schritt f) um regenerativ hergestellten elektrischen Strom (Elektrizität). Dieser regenerativ hergestellte elektrische Strom wird vorzugsweise direkt oder in unmittelbarer Nähe der Durchführung des erfindungsgemäßen Verfahrens erzeugt. Der elektrische Strom kann beispielsweise aus Wasserkraft, Windenergie, solarer Strahlung (Sonnenenergie), Erdwärme (Geothermie), Biogas, Bioethanol, Holz oder durch die Gezeiten gewonnen werden. Die zur Gewinnung des elektrischen Stroms notwendigen Maschinen und Apparate sind ebenfalls bekannt. Es kann sich dabei um Wasserturbinen, Windanlagen, Fotovoltaik, Solarthermie, Verbrennungsmaschinen oder Turbinen, bei deren Brennstoff es sich um Biomasse handelt, stammen. Vorzugsweise wird der in Schritt f) des erfindungsgemäßen Verfahrens eingesetzte elektrische Strom durch Wasserkraft, Windenergie oder solare Strahlung erzeugt. Da dieser elektrische Strom prinzipiell auch aus von nachwachsenden Rohstoffen gewonnener Biomasse, insbesondere Biogas, stammen kann, ist es auch denkbar, dass das in einer Ausführungsform der vorliegenden Erfindung parallel oder zeitversetzt hergestellte Biogas in einem nachfolgenden Schritt verstromt wird und dieser Strom, für den aktuell keine Verwendung besteht, im Rahmen des erfindungsgemäßen Verfahrens in Wasserstoff umgewandelt wird, der erfindungsgemäß gespeichert und gelagert wird.

Wie vorstehend bereits erwähnt, wird in Schritt f) (in der Regel) neben Wasserstoff zusätzlich Sauerstoff erzeugt. Vorzugsweise wird in Schritt f) zusätzlich Sauerstoff erzeugt und dieser Sauerstoff wird in der Vorrichtung (V2) gemäß Schritt b) gemeinsam mit Wasserstoff in elektrischen Strom und gegebenenfalls Wärme umgewandelt, wobei der Sauerstoff vor seiner Verwendung in Schritt b) gegebenenfalls zwischengespeichert wird. Vorrichtungen zum Zwischenspeichern von Sauerstoff sind dem Fachmann bekannt.

In einer Ausführungsform umfasst das erfindungsgemäße Verfahren die Schritte a), b) und c). Bei dieser Ausführungsform wird der optionale Schritt d) nicht durchgeführt. In einer anderen bevorzugten Ausführungsform umfasst das erfindungsgemäße Verfahren die Schritte a), b) und c) sowie zusätzlich den Schritt d) sowie gegebenenfalls den Schritt e). In diesen Ausführungsformen werden die erfindungsgemäßen Schritte a), b) und c) vorzugsweise in räumlicher und/oder zeitlicher Nähe voneinander durchgeführt, während sie hingegen vorzugsweise räumlich und/oder zeitlich getrennt von den Schritten d) und/oder e) durchgeführt werden. Eine räumliche und/oder zeitliche Nähe zwischen der Durchführung der beiden Verfahrensschritte d) und e) ist insbesondere dann vorteilhaft, wenn ein Wärmetransfer zwischen diesen beiden Schritten stattfindet.

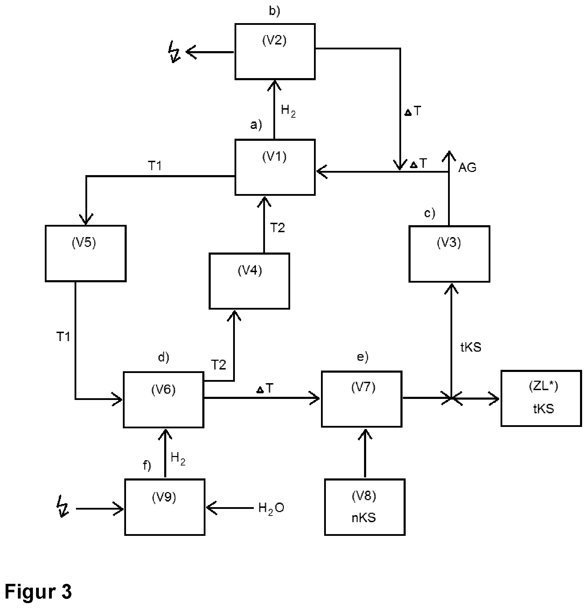

Figur 3 zeigt eine besonders bevorzugte Ausführungsform des erfindungsgemäßen Verfahrens unter Berücksichtigung der vorstehend beschriebenen Schritte a), b), c), d), e) und f). In Zusammenhang mit der Durchführung der beiden Schritte a) und b) in Figur 3 wird zunächst auf die beiden Figuren 1 und 2 sowie die zugehörigen Ausführungen verwiesen. Zur Durchführung der jeweiligen Schritte c) d), e) und f) wird auf die vorstehende Beschreibung verwiesen. Die Schritte e) und/oder f) können in dieser Ausführungsform gegebenenfalls auch weggelassen werden, vorzugsweise werden aber beide Schritte e) und f) durchgeführt.

Zusätzlich zu den beiden Figuren 1 und 2 ist in der besonders bevorzugten Ausführungsform gemäß Figur 3 mitberücksichtigt, dass gemäß Schritt d) in der Vorrichtung (V6), die vorzugsweise ein chemischer Reaktor ist, Wasserstoff mit dem Schritt a) erhaltenen Träger (T1), vorzugsweise ist der Träger (T1) ein Wasserstoffträger (LOHC), in Kontakt gebracht, wodurch der Wasserstoff gespeichert wird, vorzugsweise durch chemische Bindung an den Träger (T1), insbesondere durch Hydrierung, wodurch ein mit Wasserstoff beladener Träger (T2) erzeugt wird. Der mit Wasserstoff beladene Träger (T2) kann in der Vorrichtung (V6) verbleiben, vorzugsweise wird der Träger (T2) jedoch zur Lagerung in eine separate Vorrichtung überführt. Die einzelnen Schritte und/oder das gesamte Verfahren kann sowohl batchweise als auch kontinuierlich betrieben werden. Die bei der Speicherung des Wasserstoffs in Schritt d) freigesetzte Wärme wird nach Schritt e) transportiert.

In Schritt e) erfolgt die Einspeisung der in Schritt d) freigesetzten Wärme in eine Vorrichtung (V7), um in der Vorrichtung (V7) Klärschlamm zu trocknen. Die Vorrichtung (V7) umfasst in der Regel mindestens einen Klärschlammtrockner, vorzugsweise ausgewählt aus einem Bandtrockner, einem Trommeltrockner oder einem Scheibentrockner. Der nasse Klärschlamm (nKS) wird ebenfalls in die Vorrichtung (V7) eingespeist, wo dieser durch Wärmezufuhr unter Erhalt von getrocknetem Klärschlamm getrocknet wird. Der getrocknete Klärschlamm (tKS) kann gegebenenfalls in der Vorrichtung (V7) verbleiben, vorzugsweise wird der getrocknete Klärschlamm (tKS) jedoch weiterverarbeitet und/oder eine andere Vorrichtung transportiert. Die Vorrichtungen (V6) und (V7) befinden sich vorzugsweise in räumlicher Nähe zueinander, beispielsweise auf demselben Betriebsgelände.

Zusätzlich ist in der bevorzugten Ausführungsform gemäß Figur 3 mitberücksichtigt, dass sich der nasse Klärschlamm (nKS) zunächst vorzugsweise in einer separaten Vorrichtung (V8) befindet, bevor er in die Vorrichtung (V7) eingespeist wird.

In Anschluss an Schritt e) wird der getrocknete Klärschlamm (tKS) in eine andere Vorrichtung (V3) transportiert und dort gemäß Schritt c) weiterverarbeitet. In der Vorrichtung (V3) erfolgt die Verbrennung des getrockneten Klärschlamms. Bei der Verbrennung des getrockneten Klärschlamms entstehen Abgase (AG), die aus der Vorrichtung (V3) ausgeleitet werden. Die bei der Verbrennung des getrockneten Klärschlamms ebenfalls entstehende Wärme wird zur Freisetzung von Wasserstoff aus einem mit Wasserstoff beladenen Träger (T2) gemäß Schritt a) verwendet.

Die Vorrichtungen (V3) und (V7) befinden sich vorzugsweise in räumlicher Distanz zueinander, beispielsweise in einer Entfernung von 10-50 km. Letztendlich kann die räumliche Distanz zwischen den beiden Vorrichtungen (V7) und (V3) beliebig groß sein. Weiterhin werden die Schritte e) und c) vorzugsweise auch zeitlich getrennt voneinander durchgeführt, beispielsweise in einem Abstand von mindestens einem Monat, vorzugsweise von mindestens drei Monaten. Letztendlich kann der zeitliche Abstand zwischen der Durchführung dieser beiden Schritte beliebig groß sein. Gegebenenfalls kann der getrocknete Klärschlamm (tKS) in einer separaten Vorrichtung, beispielsweise in einem Zwischenlager in Form eines Tanks, aufbewahrt werden, nachdem Schritt e) beendigt ist und bevor Schritt c) durchgeführt wird.

Weiterhin ist in Figur 3 gezeigt, dass der getrocknete Klärschlamm (tKS) vorzugsweise in einer separaten Vorrichtung (ZL*), beispielsweise in einem Zwischenlager in Form eines Tanks, aufbewahrt werden kann, nachdem Schritt e) beendigt ist und bevor Schritt c) durchgeführt wird. Der Einsatz einer solchen separaten Vorrichtung (ZL*) ist jedoch auch in dieser besonders bevorzugten Ausführungsform nicht zwingend, sondern optional. Der Einsatz einer solchen separaten Vorrichtung (ZL*) bietet sich insbesondere dann an, sofern ein längerer Zeitraum zwischen der Durchführung der beiden Verfahrensschritte e) und c) liegt und/oder eine sehr große Menge an getrocknetem Klärschlamm (tKS) in Schritt c) verbrannt werden soll, was aus Kapazitätsgründen in der Vorrichtung (V3) einen erhöhten Lagebedarf erfordert.

In dieser Ausführungsform findet zusätzlich eine Lagerung des zurückgewonnenen, mit Wasserstoff beladenen Trägers (T2) in einer Vorrichtung (V4) sowie eine Lagerung des Trägers (T1) in einer separaten Vorrichtung (V5) statt. Die jeweiligen Lagerungen können zeitversetzt oder (zumindest teilweise) überschneidend durchgeführt werden, alternativ ist auch eine Lagerung der Träger (T1) und (T2) in einer einzigen Vorrichtung (V4) denkbar.

Gegebenenfalls kann auf die jeweiligen Lagerungen, insbesondere in den Vorrichtungen (V4) und/oder (V5), ganz oder zumindest teilweise auch verzichtet werden. Bei dieser Fallkonstellation würde beispielsweise der Schritt d) (relativ direkt) im Anschluss an die Durchführung von Schritt a) erfolgen und/oder der Schritt a) (relativ direkt) im Anschluss an die Durchführung von Schritt d) erfolgen. Sofern eine räumliche Distanz zwischen den Vorrichtungen (V1) einerseits und (V6) andererseits vorhanden ist, muss die Zeit für den benötigten Transport beispielsweise des wiedergewonnenen, mit Wasserstoff beladenen Trägers (T2) mitberücksichtigt werden, so dass Schritt a) erst mit einer zeitlichen Verzögerung durchgeführt werden kann. Ebenso kann der Transport des mit Wasserstoff beladenen Trägers (T2) in einer geeigneten Transportvorrichtung, beispielsweise ein Lkw oder ein Eisenbahn-Tankwagen, mit einer Zwischenlagerung des mit Wasserstoff beladenen Trägers (T2) verknüpft werden, indem die entsprechende Transportvorrichtung, die mit dem Träger (T2) beladen ist, für einen bestimmten Zeitraum abgestellt wird. Sinngemäßes gilt selbstverständlich für die Überführung des Trägers (T1) aus Schritt a) nach Schritt d).

Ebenso ist es im Rahmen der vorliegenden Erfindung möglich, dass beispielsweise bei mindestens einfachem durchlaufen des Zyklus bezüglich der Schritte a) und d) sowie gegebenenfalls der Schritte b) und/oder f) die Durchführung der entsprechend mehrfach durchgeführten Schritte in unterschiedlichen Vorrichtungen erfolgt. Beispielsweise wird Wasserstoff gemäß Schritt a) in einer ersten Vorrichtung (V1) freigesetzt, weil an einem ersten Standort Strom gemäß Schritt b) erzeugt werden soll. Der in Schritt a) freigesetzte Träger (T1) wird nach gegebenenfalls erfolgter Zwischenlagerung in die Vorrichtung (V6) überführt, um dort gemäß Schritt d) den Träger (T1) wieder mit Wasserstoff und Erhalt des Trägers (T2) zu beladen. Besagter mit Wasserstoff beladener Träger (T2) wird anschließend nach gegebenenfalls erfolgter Zwischenlagerung in eine zweite Vorrichtung (V1) überführt, um dort erneut Wasserstoff aus dem Träger (T2) gemäß Schritt a) freizusetzen. Allerdings befindet sich die zweite Vorrichtung (V1) an einem anderen Ort als die erste Vorrichtung (V1), weil an einem anderen Standort Strombedarf besteht. Diese Standort-unabhängige Durchführung der einzelnen Verfahrensschritte gilt selbstverständlich für alle anderen Schritte/Vorrichtungen des erfindungsgemäßen Verfahrens sinngemäß.

Die Vorrichtungen (V1) und (V2) sowie gegebenenfalls (V3) einerseits sowie die Vorrichtungen (V6) und (V9) sowie gegebenenfalls (V7) andererseits befinden sich vorzugsweise in räumlicher Distanz zueinander, beispielsweise in einer Entfernung von 10-50 km. Weiterhin werden die Schritte e) und c) und/oder die Schritte a) und d) vorzugsweise auch zeitlich getrennt voneinander durchgeführt, beispielsweise in einem Abstand von mindestens einem Monat, vorzugsweise von mindestens drei Monaten.

Im Gegensatz dazu ist es in dieser Ausführungsform bevorzugt, dass sich die Vorrichtungen (V1) und (V2) sowie gegebenenfalls (V3) und/oder die Vorrichtungen (V6) und (V9) sowie gegebenenfalls (V7) in räumlicher Nähe zueinander befinden, beispielsweise auf demselben Betriebsgelände oder auf einem benachbarten Betriebsgelände. Ebenso werden die Schritte d) und e) einerseits und/oder die Schritte c) und a) andererseits in zeitlicher Nähe zueinander, vorzugsweise parallel, durchgeführt.

In einer weiteren bevorzugten Ausführungsform der vorliegenden Erfindung wird das Verfahren in einer Anlage durchgeführt, die weiterhin zur Erzeugung von Biogas eingesetzt werden kann. Die Erzeugung von Biogas kann dabei parallel oder zeitversetzt zum erfindungsgemäßen Verfahren zur Erzeugung von Wasserstoff bzw. bei der gegebenenfalls nachfolgend durchgeführten Erzeugung von elektrischem Strom und gegebenenfalls von Wärme durchgeführt werden. Vorzugsweise wird diese Ausführungsform durchgeführt, wenn das Biogas in räumlicher und/oder zeitlicher Nähe zur Durchführung des optionalen Schritt d) bzw. zur Vorrichtung (V6) erfolgt und/oder zur Durchführung des optionalen Schritt e) im Zusammenhang mit dem Trocknen von Klärschlamm in der Vorrichtung (V7). Ebenso ist es denkbar, dass die Erzeugung von Biogas nur für ein bestimmtes Zeitintervall parallel zum Verfahren zur Erzeugung von Wasserstoff durchgeführt wird.

Vorzugsweise werden die Erzeugung von Biogas einerseits und Verfahren zur Erzeugung von Wasserstoff oder die Erzeugung von elektrischem Strom sowie gegebenenfalls von Wärme andererseits parallel auf derselben Anlage durchgeführt.

Weiterhin ist es bevorzugt, dass die Erzeugung von Biogas in einem separaten Fermenter durchgeführt wird, der nicht Bestandteil der Vorrichtung (V4) zur Lagerung des mit Wasserstoff beladenen Trägers (T2) ist.

Weiterhin ist es bevorzugt, dass der in Schritt f) erzeugte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert.

Weiterhin ist es bevorzugt, dass der in Schritt a) freigesetzte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert.

Weiterhin ist es bevorzugt, dass der in Schritt f) zusätzlich erzeugte Sauerstoff teilweise und/oder für eine bestimmte Zeitspanne zur Erzeugung von Biogas eingesetzt wird, wobei der Sauerstoff vorzugsweise in einen separaten Fermenter oder Nachgärer eingeleitet wird. Diese Ausführungsform ist deswegen vorteilhaft, weil der bei der Elektrolyse freiwerdende Sauerstoff einem Biogasprozess im Normalbetrieb zugegeben werden kann, um die Qualität des Biogases zu erhöhen. Die Zugabe von Sauerstoff anstelle von Umgebungsluft zur Belüftung des Gärprozesses vermeidet einen erhöhten Stickstoffgehalt im später erhaltenen Biogas.

Weiterhin ist es bevorzugt, dass erzeugtes Biogas, insbesondere Methan, und in den Schritten a) und/oder f) erzeugter oder freigesetzter Wasserstoff miteinander vermischt werden, um sie gemeinsam nach Schritt b) in die Vorrichtung (V2) zu überführen, wo sie in elektrischen Strom und gegebenenfalls in Wärme umgewandelt werden.

Die in den vorgenannten Optionen erwähnten Zeitspannen sind prinzipiell beliebig. Es kann sich dabei um eine oder mehrere Stunden, einen oder mehrere Tage oder sogar um noch längere Zeiträume, wie Wochen und Monate, handeln. Dies hängt ganz vom jeweiligen Bedarf der Erzeugung von Biogas, der Speicherung von Wasserstoff und/oder der Erzeugung von elektrischem Strom ab.

Ein weiterer (zweiter) Gegenstand der vorliegenden Erfindung betrifft ein Verfahren zur Erzeugung von elektrischem Strom und gegebenenfalls von Wärme umfassend die nachfolgenden Schritte a) und b):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird,
b) Überführung des in Schritt a) erhaltenen Wasserstoffs in eine Vorrichtung (V2), wobei in der Vorrichtung (V2) Wasserstoff in elektrischen Strom und gegebenenfalls in Wärme umgewandelt wird.

Die vorstehend beschriebenen Verfahrensschritte a) und b) im Zusammenhang mit dem Verfahren zur Erzeugung von elektrischem Strom und gegebenenfalls von Wärme werden sinngemäß durchgeführt entsprechend dem ersten Erfindungsgegenstand, also dem Verfahren zur Erzeugung von Wasserstoff. Besonders bevorzugt der zweite Erfindungsgegenstand als ein Verfahren durchgeführt, das die vorstehend beschriebenen Verfahrensschritte a), b) und c) umfasst.

Weiterhin können im Rahmen dieses zweiten Erfindungsgegenstandes (also dem Verfahren zur Erzeugung von elektrischem Strom und gegebenenfalls von Wärme) alle sonstigen Verfahrensschritte des ersten Erfindungsgegenstandes, insbesondere die Verfahrensschritte c) bis f) sinngemäß durchgeführt werden. Dies gilt auch für die entsprechenden bevorzugten, mehr bevorzugten, noch mehr bevorzugten oder besonders bevorzugten Definitionen sämtlicher Verfahrensschritte des ersten Erfindungsgegenstandes.

## Patentansprüche

1. Verfahren zur Erzeugung von Wasserstoff, umfassend den folgenden Schritt a):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird.

2. Verfahren gemäß Anspruch 1, **dadurch gekennzeichnet, dass** nach Schritt a) ein Schritt b) durchgeführt wird:
b) Überführung des in Schritt a) erhaltenen Wasserstoffs in eine Vorrichtung (V2), wobei in der Vorrichtung (V2) Wasserstoff in elektrischen Strom und gegebenenfalls in Wärme umgewandelt wird.

3. Verfahren gemäß Anspruch 2, **dadurch gekennzeichnet, dass**
i) die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor, ist, und/oder
ii) die gegebenenfalls in Schritt b) erzeugte Wärme nach Schritt a) zurückgeleitet wird.

4. Verfahren gemäß einem der Ansprüche 1 bis 3, **dadurch gekennzeichnet, dass** vor Schritt a) ein Schritt c) durchgeführt wird:
c) Verbrennen vom getrockneten Klärschlamm in einer Vorrichtung (V3).

5. Verfahren gemäß einem der Ansprüche 1 bis 4, **dadurch gekennzeichnet, dass** in Schritt c) bei der Klärschlammverbrennung Wärme freigesetzt wird, wobei
i) die Wärme aus dem Abgas der Verbrennungsanlage abgegeben wird, und/oder
ii) zumindest eine Teilmenge der freigesetzten Wärme, vorzugsweise unter Verwendung eines Heizbündels und/oder eines Wärmetauschers, nach Schritt a) überführt wird, um Wasserstoff aus dem Träger (T2) freizusetzen.

6. Verfahren gemäß einem der Ansprüche 1 bis 5, **dadurch gekennzeichnet, dass** der in Schritt a) eingesetzte mit Wasserstoff beladene Träger (T2) aus einem Träger (T1) erhalten wird, wobei der Träger (T1) mit Wasserstoff in Kontakt gebracht wird, vorzugsweise dient der mit Wasserstoff beladene Träger (T2) als Wasserstoffspeicher.

7. Verfahren gemäß einem der Ansprüche1 bis 6, **dadurch gekennzeichnet, dass**
i) vor Durchführung von Schritt a) der mit Wasserstoff beladene Träger (T2) in einer Vorrichtung (V4) gelagert wird, vorzugsweise ist die Vorrichtung (V4) ausgewählt aus einem Tank, einen Fermenter oder einem Behältnis, das durch Umrüsten einer Biogasanlage, insbesondere durch Umrüstung eines Fermenters, erhalten wurde oder das Bestandteil einer Biogasanlage ist, und/oder
ii) Wasserstoff chemisch oder physisorptiv, vorzugsweise chemisch, an den Träger (T1) gebunden wird, und/oder
iii) der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol, ist, und/oder
iv) der mit Wasserstoff beladene Träger (T2) durch zumindest teilweise oder vollständige Hydrierung, vorzugsweise durch vollständige Hydrierung, mit Wasserstoff aus dem Träger (T1) erhalten wird.

8. Verfahren gemäß einem der Ansprüche 1 bis 7, **dadurch gekennzeichnet, dass**
i) bei der Freisetzung von Wasserstoff gemäß Schritt a) zusätzlich der Träger (T1) wiedergewonnen wird, oder
ii) bei der Freisetzung von Wasserstoff gemäß Schritt a) zusätzlich der Träger (T1) wiedergewonnen wird und anschließend der Träger (T1) in einer separaten Vorrichtung (V5) oder in der Vorrichtung (V4) gelagert wird, und/oder
iii) Schritt a) in einer Vorrichtung (V1) durchgeführt wird, die vorzugsweise ein chemischer Reaktor ist.

9. Verfahren gemäß Anspruch 8, **dadurch gekennzeichnet, dass** mit dem bei der Freisetzung des Wasserstoffs erhaltene Träger (T1) ein Schritt d) ausgeführt wird, wobei
d) Inkontaktbringen eines Trägers (T1) mit Wasserstoff zur Speicherung des Wasserstoffs unter Erhalt eines mit Wasserstoff beladenen Trägers (T2) sowie Freisetzung von Wärme,

10. Verfahren gemäß Anspruch 9, **dadurch gekennzeichnet, dass** nach Schritt d) ein Schritt e) durchgeführt wird, wobei
e) Einspeisung der in Schritt d) freigesetzten Wärme in eine Vorrichtung (V7), um in der Vorrichtung (V7) Klärschlamm zu trocknen.

11. Verfahren gemäß Anspruch 10, **dadurch gekennzeichnet, dass** in Schritt e)
i) die Vorrichtung (V7) mindestens einen Klärschlammtrockner umfasst, vorzugsweise ausgewählt aus einem Bandtrockner, einem Trommeltrockner oder einem Scheibentrockner, und/oder
ii) die in Schritt d) freigesetzte Wärme über eine Rohrleitung oder ein Rohrleitungssystem in die Vorrichtung (V7) geleitet wird, und/oder
iii) die in Schritt d) freigesetzte Wärme durch einen Wärmetauscher geleitet wird, um die Trocknungsluft in der Vorrichtung (V7), insbesondere in einem Bandtrockner, aufzuheizen, und/oder
iv) die Vorrichtung (V7) mindestens zwei Bereiche mit unterschiedlichen Temperaturzonen aufweist.

12. Verfahren gemäß Anspruch 10 oder 11, **dadurch gekennzeichnet, dass**
i) Schritt d) und Schritt e) in räumlicher Nähe durchgeführt werden, vorzugsweise befinden sich die jeweiligen Vorrichtungen zur Durchführung der beiden Schritte d) und e) auf demselben Betriebsgelände, und/oder
ii) die Vorrichtung (V7) umfasst a) eine Niedertemperaturzone (Z1), die bei einem Temperaturniveau von 50 bis 90 °C, vorzugsweise bei 60 bis 80 °C, insbesondere bei 70 °C, betrieben wird, und b) eine Hochtemperaturzone (Z2), die bei einem Temperaturniveau von 100 bis 180 °C, vorzugsweise bei 130 bis 150 °C, insbesondere bei 140 °C, betrieben wird, und/oder
iii) der Klärschlamm in der Vorrichtung (V7) bis zu einem Trocknungsgrad von mindestens 75 %, vorzugsweise bei mindestens 85 %, insbesondere bei 95 bis 100 % (Volltrocknung) getrocknet wird, und/oder
iv) der Klärschlamm zunächst entwässert wird und mit einem Trocknungsgrad von 20 bis 30 % vorliegt, bevor er in die Vorrichtung (V7) zur Trocknung eingespeist wird, und/oder
v) der Klärschlamm mit einer Dickstoffpumpe in die Vorrichtung (V7) zur Trocknung eingespeist wird, und/oder
vi) der Klärschlamm vor der Trocknung in der Vorrichtung (V7) in einer Vorrichtung (V8) gelagert wird, vorzugsweise ist die Vorrichtung (V8) ausgewählt aus einem Tank, einen Fermenter oder einem Behältnis, das durch Umrüsten einer Biogasanlage, insbesondere durch Umrüstung eines Fermenters, erhalten wurde oder das Bestandteil einer Biogasanlage ist.

13. Verfahren gemäß einem der Ansprüche 1 bis 12, **dadurch gekennzeichnet, dass**
i) der in Schritt e) getrocknete Klärschlamm in die Vorrichtung (V3) überführt wird, um gemäß Schritt c) getrockneten Klärschlamm in der Vorrichtung (V3) zu verbrennen, und/oder
ii) vor Durchführung von Schritt c) der getrocknete Klärschlamm zwischengelagert wird, und/oder
iii) Schritt e) und Schritt c) räumlich getrennt voneinander durchgeführt werden, vorzugsweise in einer Entfernung von mindestens 5 km, insbesondere 10 bis 50 km, und/oder
iv) Schritt e) und Schritt c) zeitlich getrennt voneinander durchgeführt werden, vorzugsweise wird Schritt c) mindestens einen Monat, besonders bevorzugt mindestens drei Monate, nach Schritt e) durchgeführt.

14. Verfahren gemäß einem der Ansprüche 9 bis 13, **dadurch gekennzeichnet, dass** vor Schritt d) ein Schritt f) durchgeführt wird:
f) Erzeugung von Wasserstoff in einer Vorrichtung (V9), vorzugsweise in einem Elektrolyseur, unter Verwendung von Wasser und elektrischem Strom.

15. Verfahren gemäß einem der Ansprüche 1 bis 14, **dadurch gekennzeichnet, dass**
i) Schritt d) in einer separaten Vorrichtung (V6) durchgeführt wird, die vorzugsweise ein chemischer Reaktor ist, und/oder
ii) die Vorrichtung (V4) zwei räumlich getrennte Bereiche aufweist, wobei ein erster Bereich zur Lagerung des mit Wasserstoff beladenen Trägers (T2) verwendet wird und ein zweiter Bereich zur Lagerung des Trägers (T1) verwendet wird, und/oder
iii) die Vorrichtung (V4) in ihrem Inneren mit einer Schutzauskleidung versehen wird, bevor die Lagerung des Trägers (T2) und gegebenenfalls des Trägers (T1) durchgeführt wird, und/oder
iv) in die Vorrichtung (V4) eine schwimmende Trennwand eingebaut ist, wodurch der Träger (T1) getrennt von dem mit Wasserstoff beladenen Träger (T2) gelagert wird, und/oder
v) die Vorrichtung (V4) Bestandteil einer Biogasanlage war und von allen Substanzen gereinigt war, die sich infolge der Erzeugung von Biogas in ihrem Inneren oder gegebenenfalls in ihren Zuleitungen befunden haben, bevor die Lagerung des Trägers (T2) und gegebenenfalls des Trägers (T1) durchgeführt wird.

16. Verfahren gemäß einem der Ansprüche 1 bis 15, **dadurch gekennzeichnet, dass** das Verfahren in einer Anlage durchgeführt wird, die weiterhin zur Erzeugung von Biogas eingesetzt werden kann.

17. Verfahren gemäß Anspruch 16, **dadurch gekennzeichnet, dass**
i) die Erzeugung von Biogas einerseits und das Speichern und Lagern von Wasserstoff oder die Erzeugung von elektrischem Strom sowie gegebenenfalls von Wärme andererseits parallel auf derselben Anlage durchgeführt werden, und/oder
ii) die Erzeugung von Biogas in einem separaten Fermenter durchgeführt wird, der nicht Bestandteil der Vorrichtung (V4) zur Lagerung des mit Wasserstoff beladenen Trägers (T2) ist.

18. Verfahren zur Erzeugung von elektrischem Strom und gegebenenfalls von Wärme umfassend die nachfolgenden Schritte a) und b):
a) Freisetzung von Wasserstoff aus einem mit Wasserstoff beladenen Träger (T2), wobei zumindest eine Teilmenge der zur Wasserstofffreisetzung erforderlichen Energie durch die Verbrennung von Klärschlamm gewonnen wird,
b) Überführung des in Schritt a) erhaltenen Wasserstoffs in eine Vorrichtung (V2), wobei in der Vorrichtung (V2) Wasserstoff in elektrischen Strom und gegebenenfalls in Wärme umgewandelt wird.

## Claims

1. Process for the production of hydrogen, comprising the following step a):
a) release of hydrogen from a hydrogen-loaded carrier (T2), wherein at least a part of the energy required for the hydrogen release is obtained by the incineration of sewage sludge.

2. Process according to claim 1, **characterized in that** after step a) a step b) is carried out:
b) transfer of the hydrogen obtained in step a) to a device (V2), wherein in the device (V2) hydrogen is converted into electric current and, optionally, into heat.

3. Method according to claim 2, **characterized in that**
i) the device (V2) is part of a combined heat and power plant, a gas engine, a fuel cell, an internal combustion engine, an internal combustion engine with connected generator, a turbine or a hydrogen turbine with connected generator, preferably a gas engine, and/or
ii) the heat generated in step b), if any, is recycled to step a).

4. Process according to any one of claims 1 to 3, **characterized in that** step a) is preceded by step c):
c) incineration from the dried sewage sludge in a device (V3).

5. Process according to any one of claims 1 to 4, **characterized in that** in step c) heat is released during sewage sludge incineration, wherein
i) the heat is released from the exhaust gas of the incineration plant, and/or
ii) at least part of the released heat is transferred, preferably using a heating bundle and/or a heat exchanger, to step a) to release hydrogen from the carrier (T2).

6. Process according to any one of claims 1 to 5, **characterized in that** the hydrogen-loaded carrier (T2) used in step a) is obtained from a carrier (T1), the carrier (T1) being brought into contact with hydrogen, preferably the hydrogen-loaded carrier (T2) serves as hydrogen storage.

7. Process according to any one of claims 1 to 6, **characterized in that**
i) before carrying out step a), the hydrogen-loaded carrier (T2) is stored in a device (V4), preferably the device (V4) is selected from a tank, a fermenter or a container which has been obtained by converting a biogas plant, in particular by converting a fermenter, or which is a component of a biogas plant, and/or
ii) hydrogen is chemically or physisorptically, preferably chemically, bound to the carrier (T1), and/or
iii) the carrier (T1) is an unsaturated compound, preferably a compound with at least one carbon-carbon double bond, more preferably an aromatic or hetero-aromatic compound, even more preferably an N-alkylcarbazole, toluene, dibenzyltoluene, benzyltoluene, naphthalene or an azaborine, particularly preferably dibenzyltoluene, and/or
iv) the hydrogen-loaded carrier (T2) is obtained by at least partial or complete hydrogenation, preferably complete hydrogenation, with hydrogen from the carrier (T1).

8. Process according to any one of claims 1 to 7, **characterized in that**
i) during the release of hydrogen according to step a), the carrier (T1) is additionally recovered, or
ii) during the release of hydrogen according to step a), additionally the carrier (T1) is recovered and subsequently the carrier (T1) is stored in a separate device (V5) or in the device (V4), and/or
iii) step a) is carried out in a device (V1), which is preferably a chemical reactor.

9. Process according to claim 8, **characterized in that** a step d) is carried out with the carrier (T1) obtained during the release of the hydrogen, wherein
d) a carrier (T1) is brought into contact with hydrogen to store the hydrogen while obtaining a hydrogen-loaded carrier (T2), and wherein heat is released.

10. Process according to claim 9, **characterized in that** after step d) a step e) is carried out, wherein
e) the heat released in step d) is fed into a device (V7) to dry sewage sludge in the device (V7).

11. Process according to claim 10, **characterized in that** in step e)
i) the apparatus (V7) comprises at least one sewage sludge dryer, preferably selected from a belt dryer, a drum dryer or a disc dryer, and/or
ii) the heat released in step d) is conducted into the apparatus (V7) via a pipeline or a piping system, and/or
iii) the heat released in step d) is conducted through a heat exchanger to heat the drying air in the apparatus (V7), in particular in a belt dryer, and/or
iv) the device (V7) has at least two areas with different temperature zones.

12. Process according to claim 10 or 11, **characterized in that**
i) step d) and step e) are carried out in spatial proximity, preferably the respective devices for carrying out the two steps d) and e) are located on the same premises, and/or
ii) the apparatus (V7) comprises a) a low-temperature zone (Z1) which is operated at a temperature level of 50 to 90 °C, preferably at 60 to 80 °C, in particular at 70 °C, and b) a high-temperature zone (Z2) which is operated at a temperature level of 100 to 180 °C, preferably at 130 to 150 °C, in particular at 140 °C, and/or
iii) the sewage sludge is dried in the apparatus (V7) to a drying degree of at least 75%, preferably at least 85%, in particular at 95 to 100% (full drying), and/or
iv) the sewage sludge is first dehydrated and is present with a degree of dryness of 20 to 30 % before it is fed into the apparatus (V7) for drying, and/or
v) the sewage sludge is fed into the drying device (V7) by means of a thick matter pump, and/or
vi) the sewage sludge is stored in a device (V8) before drying in the device (V7), preferably the device (V8) is selected from a tank, a fermenter or a container which has been obtained by converting a biogas plant, in particular by converting a fermenter, or which is a component of a biogas plant.

13. Process according to any one of claims 1 to 12, **characterized in that**
i) the sewage sludge dried in step e) is transferred to the apparatus (V3) for, according to step c), incinerating sewage sludge dried in the apparatus (V3), and/or
ii) before carrying out step c), the dried sewage sludge is temporarily stored, and/or
iii) step e) and step c) are carried out spatially separated from each other, preferably at a distance of at least 5 km, in particular 10 to 50 km, and/or
iv) step e) and step c) are carried out separately in time, preferably step c) is carried out at least one month, particularly preferably at least three months, after step e).

14. Process according to any one of claims 9 to 13, **characterized in that** step d) is preceded by step f):
(f) generation of hydrogen in a device (V9), preferably in an electrolyzer, using water and electric current.

15. Process according to any one of claims 1 to 14, **characterized in that**
i) step d) is carried out in a separate apparatus (V6), which is preferably a chemical reactor, and/or
ii) the apparatus (V4) comprises two spatially separated areas, a first area being used for storing the carrier (T2) loaded with hydrogen and a second area being used for storing the carrier (T1), and/or
iii) the device (V4) is provided with a protective lining in its interior before the storage of the carrier (T2) and optionally the carrier (T1) is carried out, and/or
iv) a floating partition is installed in the apparatus (V4), whereby the carrier (T1) is stored separately from the hydrogen-loaded carrier (T2), and/or
v) the device (V4) has been part of a biogas plant and has been cleaned of all substances that have been present inside it or eventually in its feed lines as a result of the production of biogas before the storage of the carrier (T2) and eventually of the carrier (T1) is carried out.

16. Process according to any one of claims 1 to 15, **characterized in that** the process is carried out in a plant which can still be used for the production of biogas.

17. Process according to claim 16, **characterized in that**
i) the production of biogas on the one hand and the storage and stockage of hydrogen or the production of electric current as well as eventually of heat on the other hand are carried out in parallel on the same installation, and/or
ii) the production of biogas is carried out in a separate fermenter which is not part of the device (V4) for storing the carrier (T2) loaded with hydrogen.

18. Process for the production of electric currant and optionally heat comprising the following steps a) and b):
a) release of hydrogen from a hydrogen-loaded carrier (T2), wherein at least part of the energy required for hydrogen release is obtained by incinerating sewage sludge,
b) transfer of the hydrogen obtained in step a) to a device (V2), wherein hydrogen is converted into electric current and optionally into heat in the device (V2).

## Revendications

1. Procédé de production d'hydrogène, comprenant l'étape a) suivante:
a) dégagement d'hydrogène à partir d'un support (T2) chargé d'hydrogène, au moins une partie de l'énergie nécessaire au dégagement d'hydrogène étant obtenue par l'incinération de boues d'épuration.

2. Procédé selon la revendication 1, **caractérisé en ce que**, après l'étape a), une étape b) est effectuée:
b) transfert de l'hydrogène obtenu à l'étape a) dans un dispositif (V2), l'hydrogène étant converti dans le dispositif (V2) en courant électrique et éventuellement en chaleur.

3. Procédé selon la revendication 2, **caractérisé en ce que**
i) le dispositif (V2) fait partie d'une centrale de cogénération, un moteur à gaz, une pile à combustible, un moteur à combustion interne, un moteur à combustion interne avec générateur raccordé, une turbine ou une turbine à hydrogène avec générateur raccordé, de préférence un moteur à gaz, et/ou
ii) la chaleur éventuellement générée à l'étape b) est recyclée vers l'étape a).

4. Procédé selon l'une des revendications 1 à 3, **caractérisé en ce qu'**une étape c) est réalisée avant l'étape a) :
c) incinération des boues d'épuration séchées dans un dispositif (V3).

5. Procédé selon l'une des revendications 1 à 4, **caractérisé en ce que**, dans l'étape c), de la chaleur est dégagée lors de l'incinération des boues d'épuration, dans laquelle
i) la chaleur est dégagée à partir des gaz d'échappement de l'incinérateur, et/ou
ii) au moins une partie de la chaleur dégagée est transférée, de préférence en utilisant un faisceau de chauffage et/ou un échangeur de chaleur, à l'étape a) pour dégager de l'hydrogène du support (T2).

6. Procédé selon l'une quelconque des revendications 1 à 5, **caractérisé en ce que** le support (T2) chargé d'hydrogène utilisé à l'étape a) est obtenu à partir d'un support (T1), le support (T1) étant mis en contact avec de l'hydrogène, de préférence le support (T2) chargé d'hydrogène sert de stockage d'hydrogène.

7. Procédé selon l'une quelconque des revendications 1 à 6, **caractérisé en ce que**
i) avant l'exécution de l'étape a), le support (T2) chargé d'hydrogène est stocké dans un dispositif (V4), de préférence le dispositif (V4) est choisi parmi un réservoir, un fermenteur ou un récipient qui a été obtenu par transformation d'une installation de biogaz, en particulier par transformation d'un fermenteur, ou qui fait partie d'une installation de biogaz, et/ou
ii) l'hydrogène est lié au support (T1) par voie chimique ou physisorptive, de préférence par voie chimique, et/ou
iii) le support (T1) est un composé insaturé, de préférence un composé ayant au moins une double liaison carbone-carbone, plus préférablement un composé aromatique ou hétéroaromatique, encore plus préférablement un N-alkylcarbazole, toluène, dibenzyltoluène, benzyltoluène, naphtalène ou une azaborine, plus préférablement dibenzyltoluène, et/ou
iv) le support (T2) chargé d'hydrogène est obtenu par hydrogénation au moins partielle ou totale, de préférence par hydrogénation totale, avec de l'hydrogène du support (T1).

8. Procédé selon l'une quelconque des revendications 1 à 7, **caractérisé en ce que**
i) lors du dégagement d'hydrogène selon l'étape a), le support (T1) est en outre récupéré, ou
ii) lors du dégagement d'hydrogène selon l'étape a), le support (T1) est en outre récupéré, puis le support (T1) est stocké dans un dispositif séparé (V5) ou dans le dispositif (V4), et/ou
iii) l'étape a) est réalisée dans un dispositif (V1) qui est de préférence un réacteur chimique.

9. Procédé selon la revendication 8, **caractérisé en ce qu'**une étape d) est réalisée avec le support (T1) obtenu lors du dégagement l'hydrogène, dans laquelle d) un support (T1) est mis en contact avec de l'hydrogène pour stocker l'hydrogène tout en obtenant un support (T2) chargé d'hydrogène, et dans laquelle de la chaleur étant dégagée.

10. Procédé selon la revendication 9, **caractérisé en ce que**, après l'étape d), on effectue une étape e), dans laquelle
e) la chaleur dégagée à l'étape d) est injectée dans un dispositif (V7) pour sécher des boues d'épuration dans le dispositif (V7).

11. Procédé selon la revendication 10, **caractérisé en ce que**, dans l'étape e)
i) le dispositif (V7) comprend au moins un séchoir de boues d'épuration, de préférence choisi parmi un séchoir à bande, un séchoir à tambour ou un séchoir à disques, et/ou
ii) la chaleur dégagée à l'étape d) est introduite dans le dispositif (V7) par l'intermédiaire d'un élément de conduit ou d'un système d'éléments de conduit, et/ou
iii) la chaleur dégagée à l'étape d) est conduite à travers un échangeur de chaleur pour chauffer l'air de séchage dans le dispositif (V7), en particulier dans un séchoir à bande, et/ou
iv) le dispositif (V7) comporte au moins deux zones ayant des températures différentes.

12. Procédé selon la revendication 10 ou 11, **caractérisé en ce que**
i) l'étape d) et l'étape e) sont réalisées à proximité géographique, de préférence les dispositifs respectifs pour la réalisation des deux étapes d) et e) sont situés sur le même site, et/ou
ii) le dispositif (V7) comprend a) une zone à basse température (Z1) qui fonctionne à un niveau de température de 50 à 90 °C, de préférence de 60 à 80 °C, en particulier de 70 °C, et b) une zone à haute température (Z2) qui fonctionne à un niveau de température de 100 à 180 °C, de préférence de 130 à 150 °C, en particulier de 140 °C, et/ou
iii) les boues d'épuration sont séchées dans le dispositif (V7) jusqu'à un degré de séchage d'au moins 75 %, de préférence d'au moins 85 %, en particulier de 95 à 100 % (séchage complet), et/ou
iv) les boues d'épuration sont d'abord déshydratées et se présentent avec un degré de séchage de 20 à 30 % avant d'être introduites dans le dispositif (V7) pour être séchées, et/ou
v) les boues d'épuration sont introduites dans le dispositif (V7) de séchage au moyen d'une pompe à matière épaisse, et/ou
vi) les boues d'épuration sont stockées dans un dispositif (V8) avant le séchage dans le dispositif (V7), de préférence le dispositif (V8) est choisi parmi un réservoir, un fermenteur ou un récipient qui a été obtenu par transformation d'une installation de biogaz, en particulier par transformation d'un fermenteur, ou qui fait partie d'une installation de biogaz.

13. Procédé selon l'une quelconque des revendications 1 à 12, **caractérisé en ce que**
i) les boues d'épuration séchées à l'étape e) sont transférées dans le dispositif (V3) pour incinérer selon l'étape c) les boues d'épuration séchées dans le dispositif (V3), et/ou
ii) avant l'exécution de l'étape c), les boues d'épuration séchées sont stockées temporairement, et/ou
iii) l'étape e) et l'étape c) sont réalisées séparément dans l'espace, de préférence à une distance d'au moins 5 km, en particulier de 10 à 50 km, et/ou
iv) l'étape e) et l'étape c) sont réalisées séparément dans le temps, de préférence l'étape c) est réalisée au moins un mois, de préférence au moins trois mois, après l'étape e).

14. Procédé selon l'une quelconque des revendications 9 à 13, **caractérisé en ce qu'**une étape f) est réalisée avant l'étape d):
f) production d'hydrogène dans un dispositif (V9), de préférence dans un électrolyseur, en utilisant de l'eau et du courant électrique.

15. Procédé selon l'une quelconque des revendications 1 à 14, **caractérisé en ce que**
i) l'étape d) est réalisée dans un dispositif séparé (V6), qui est de préférence un réacteur chimique, et/ou
ii) le dispositif (V4) comporte deux zones séparées dans l'espace, une première zone étant utilisée pour le stockage du support (T2) chargé en hydrogène et une seconde zone étant utilisée pour le stockage du support (T1), et/ou
iii) le dispositif (V4) est muni à l'intérieur d'un garnissage de protection avant de procéder au stockage du support (T2) et éventuellement du support (T1), et/ou
iv) une cloison de séparation flottante est installée dans le dispositif (V4), ce qui permet de stocker le support (T1) séparément du support (T2) chargé d'hydrogène, et/ou
v) le dispositif (V4) faisait partie d'une installation de biogaz et était nettoyé de toutes les substances qui se trouvaient à l'intérieur ou éventuellement dans ses conduites d'alimentation à la suite de la production de biogaz, avant que le stockage du support (T2) et éventuellement du support (T1) ne soit effectué.

16. Procédé selon l'une quelconque des revendications 1 à 15, **caractérisé en ce que** le procédé est mis en oeuvre dans une installation qui peut encore être utilisée pour produire du biogaz.

17. Procédé selon la revendication 16, **caractérisé en ce que**
i) la production de biogaz d'une part et le stockage et l'entreposage d'hydrogène ou la production de courant électrique ainsi qu'éventuellement de chaleur d'autre part sont effectués en parallèle sur la même installation, et/ou
ii) la production de biogaz est réalisée dans un fermenteur séparé qui ne fait pas partie du dispositif (V4) de stockage du support (T2) chargé d'hydrogène.

18. Procédé de production de courant électrique et, le cas échéant, de chaleur, comprenant les étapes a) et b) suivantes:
a) dégagement d'hydrogène à partir d'un support (T2) chargé d'hydrogène, au moins une partie de l'énergie nécessaire au dégagement d'hydrogène étant obtenue par l'incinération de boues d'épuration,
b) transfert de l'hydrogène obtenu à l'étape a) dans un dispositif (V2), l'hydrogène étant converti en courant électrique et éventuellement en chaleur dans le dispositif (V2).
